# EUROPEAN PATENT SPECIFICATION

(11) **EP 3 700 534 B1**
(45) Date of publication and mention of the grant of the patent: **06.03.2024**
(21) Application number: 18804704.7
(22) Date of filing: 25.10.2018
(51) Int. Cl.: A61K 31/5513, A61P 43/00

(54) **METHODS OF TREATING PHELAN MCDERMID SYNDROME USING FARNESYL DIBENZODIAZEPINONES**
VERFAHREN ZUR BEHANDLUNG VON PHELAN-MCDERMID-SYNDROM UNTER VERWENDUNG VON FARNESYLDIBENZODIAZEPINONEN
MÉTHODES DE TRAITEMENT DU SYNDROME DE PHELAN MCDERMID À L'AIDE DE FARNÉSYL-DIBENZODIAZÉPINONES

(30) Priority: 27.10.2017 US 201762577821 P
(43) Date of publication of application: 02.09.2020
(73) Proprietor: Amo Pharma Ltd., Wonersh GU5 0PF (GB)
(72) Inventor: SNAPE, Michael, Wonersh GU5 0PF (GB); COGRAM, Patricia, Barnet EN5 5BY (GB); DEACON, Robert, Oxford OX4 4JB (GB)
(74) Representative: Dehns
(86) International application number: PCT/IB2018/058345
(87) International publication number: WO 2019/082125

(56) References cited:
- WO-A1-2012/117334
- US-A1- 2006 276 436
- ALEXANDER KOLEVZON ET AL: "A pilot controlled trial of insulin-like growth factor-1 in children with Phelan-McDermid syndrome", MOLECULAR AUTISM, BIOMED CENTRAL LTD, LONDON, UK, vol. 5, no. 1, 12 December 2014 (2014-12-12), page 54, XP021208398, ISSN: 2040-2392, DOI: 10.1186/2040-2392-5-54
- Anonymous: "AMO-01 to Treat Adolescents and Adults With Phelan-McDermid Syndrome (PMS) and Co-morbid Epilepsy - Full Text View - ClinicalTrials.gov", , 10 April 2018 (2018-04-10), XP055548827, Retrieved from the Internet: URL:https://clinicaltrials.gov/ct2/show/NC T03493607 [retrieved on 2019-01-29]
- SNAPE MICHAEL F ET AL: "5.16 AMO-01 in the SHANK3 Knockout Transgenic Mouse Model of Phelan Mcdermid Syndrome", JOURNAL OF THE AMERICAN ACADEMY OF CHILD & ADOLESCENT PSYCHIATRY, vol. 56, no. 10, 23 October 2017 (2017-10-23), page S258, XP085245575, ISSN: 0890-8567, DOI: 10.1016/J.JAAC.2017.09.299

## Description

### BACKGROUND OF INVENTION

Phelan McDermid Syndrome (PMS) is a neurodevelopmental disorder first mentioned in the literature by Watt et al. (1985) and then described in more detail by Phelan et al. (2001).

The clinical features of PMS in humans include severe neonatal hypotonia (>97% individuals), global developmental delay (>98%), normal to accelerated growth (95%), absent to severely delayed speech (>98%), and minor dysmorphic features (Phelan 2008). Behaviorally, Phelan McDermid individuals commonly present with hyposensitivity to pain, poor eye contact, stereotypic movements, decreased social function, hyperactivity, altered sensorimotor function and aggressive behavior. The behavioral phenotype may regress with age, showing a decline in social, motor or psychiatric function (Soorya et al. 2013). Physically, PMS may be associated with minor dysmorphic features and lymphedema.

A key co-morbidity in PMS is presence of epilepsy. As many as 41% of affected individuals will have seizures (febrile and/or non-febrile) (Soorya et al. 2013). Holder and Quach (2016) also describe 46% of Phelan McDermid individuals having seizures at some point in their life, with all types of seizures presenting. The most common seizures were atypical absence seizures (90%), with other types including tonic (54%), atonic (18%), tonic-clonic (9%), and myoclonic (9%). 20% of the individuals characterized by Holder and Quach had suffered status epilepticus and 8% were diagnosed with Lennox Gastaut Syndrome. Seizures may be pharmacologically controlled or pharmaco-resistant. There is a trend towards increased seizure susceptibility with age, and seizure onset correlates with regression of functional skills (Soorya et al. 2013). Multiple anti-convulsants have been used and poly-pharmacy for seizures is common.

The disorder in humans is characterized by a chromosomal deletion at 22q13.3 that contains the *SHANK3*/*ProSAP2* gene (Bonaglia et al. 2001; Anderlid et al. 2002; Wilson et al. 2003). Consensus opinion regards the signs and symptoms of PMS as relating to haploinsufficiency of the SHANK3 protein.

Shcheglovitov et al. (2013) showed that induced pluripotent stem (IPS) cell lines derived from PMS patients have impaired post-synaptic AMPA and NMDA receptor responses that reflect fewer excitatory synapses. These deficits related to decreased levels of the longest isoform of SHANK3 protein (Shcheglovitov et al. (2013)) and were rescued by administration of insulin-like growth factor 1 (IGF-1). The authors proposed SHANK3 is required at an early stage of synapse formation, and that IGF-1 promotes maturation of synapses by triggering the loss of SHANK3 and recruitment of PSD95 protein. Bozdagi et al. (2013) also showed rescue of the phenotype of a *Shank3* knockout mouse with IGF-1. Kolevzon et al. (2014) reported that IGF-1 administration in placebo controlled conditions resulted in improvements in social, repetitive and aberrant behaviors of the *Shank3* knockout mice during the course of 12 weeks of treatment.

There has been relatively little clinical study of pharmacological interventions in PMS, although a study of the effects of intranasal oxytocin in PMS is underway in the United States (ClinicalTrials.gov identifier NCT02710084). WO2012/117334 discloses allosteric modulators of mGluR.5 for use in the treatment of Phelan McDermid syndrome.

There are no approved treatments for PMS.

Therefore means for treating PMS are needed; the invention presented herein is directed to such ends.

### BRIEF SUMMARY OF INVENTION

The present invention relates to compounds and compositions for use in methods for treating Phelan McDermid Syndrome (PMS) in subjects having the disorder or in subjects predisposed to develop the disorder. As discussed more fully below, it has been found that farnesyl dibenzodiazepinone compounds can reduce symptoms of the disorder in PMS individuals to which the compounds are administered. The scope of the invention is defined by the claims. The references to methods of treatment in the summary and detailed description of the invention in this description are to be interpreted as references to the compounds, pharmaceutical compositions and medicaments of the present invention for use in a method for treatment of the human (or animal) body by therapy.

Herein disclosed is the use of (i) at least one farnesyl dibenzodiazepinone compound or (ii) a pharmaceutical formulation comprising at least one farnesyl dibenzodiazepinone compound and a pharmaceutically acceptable carrier or diluent in the treatment of a subject having PMS or in a method of treating a subject having PMS.

Further disclosed is the use of (i) at least one farnesyl dibenzodiazepinone compound or (ii) a pharmaceutical formulation comprising at least one farnesyl dibenzodiazepinone compound and a pharmaceutically acceptable carrier or diluent in the inhibition of development of PMS in a subject predisposed to develop PMS or in a method of inhibiting development of PMS in a subject predisposed to develop PMS.

Further disclosed is a therapeutically effective amount of (i) at least one farnesyl dibenzodiazepinone compound or (ii) a pharmaceutical formulation comprising at least one farnesyl dibenzodiazepinone compound and a pharmaceutically acceptable carrier or diluent for use in the treatment of a subject having PMS or in a method of treating a subject having PMS.

Further disclosed is a therapeutically effective amount of (i) at least one farnesyl dibenzodiazepinone compound or (ii) a pharmaceutical formulation comprising at least one farnesyl dibenzodiazepinone compound and a pharmaceutically acceptable carrier or diluent for use in the inhibition of development of PMS in a subject predisposed to develop PMS or in a method of inhibiting development of PMS in a subject predisposed to develop PMS.

The invention is drawn to a therapeutically effective amount of a compound for use in the treatment or prevention of Phelan McDermid Syndrome (PMS), in a subject having PMS, wherein the compound is selected from compounds encompassed by Formula I or pharmaceutically acceptable salts thereof: 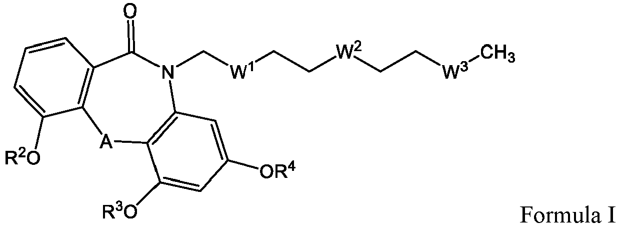 wherein,
each of W¹, W² and W³ is independently 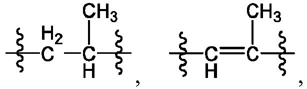 or the chain from the tricycle may terminate at W³, W² or W¹ with W³, W² or W¹ respectively being either -CH=O or -CH₂OH; A is -NH-, or -NC(O)R¹-, where R¹ is C₁₋₆ alkyl,
each of R², R³, and R⁴ is independently H.

In each of the embodiments and aspects of the invention, compound may be the compound AMO-01 or a pharmaceutically acceptable salt thereof.

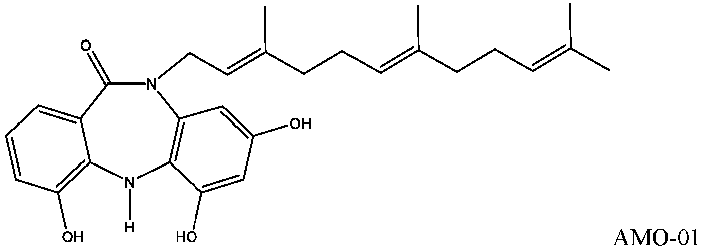

In relevant embodiments and aspects of the invention, a subject predisposed to develop PMS is a subject having a chromosomal deletion at 22q13.3.

In each of the embodiments and aspects of the invention, the therapeutically effective amount of the at least one compound of formula (I) or the pharmaceutical formulation comprising at least one compound of formula (I) and a pharmaceutically acceptable carrier or diluent is between about 0.1 ug to about 200 mg per kg of the agent per body weight of the subject.

In each of the embodiments and aspects of the invention, the at least one compound of formula (I) or the pharmaceutical formulation comprising at least one compound of formula (I) and a pharmaceutically acceptable carrier or diluent is administered to the subject via intravenous, subcutaneous, or other acceptable means.

The novel features which are believed to be characteristic of the invention, both as to its organization and method of operation, together with further objects and advantages will be better understood from the following description when considered in connection with the accompanying figures. It is to be expressly understood, however, that any description, figure, example, etc. is provided for the purpose of illustration and description only and is by no means intended to define the limits the invention which is defined by the claims.

### BRIEF DESCRIPTION OF DRAWINGS

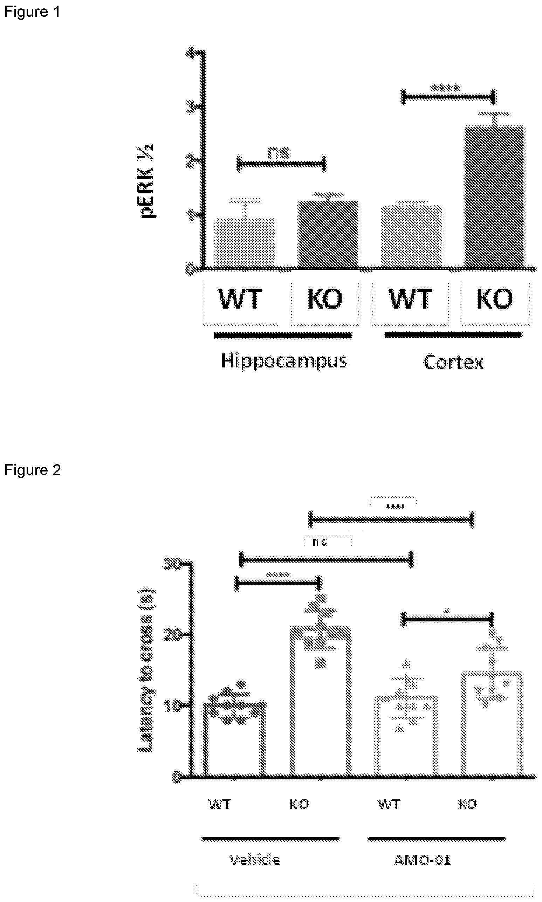

Figure 1. Activation of the Ras-ERK pathway in wild type (WT) and *Shank3b*^{*-*/*-*} knockout (KO) transgenic mice as indexed by pERK levels in hippocampus and cortex, "ns" is Not Significant; **** is p < 0.0001.
Figure 2. Anxiety as assessed by light-dark box testing in *Shank3b* knockout transgenic model of PMS after a single dose 30 mg/kg i.p. AMO-01 or vehicle administered 4 hours before testing in groups of n = 10 mice. The overall ANOVA used to analyze this experiment was significant (F = 31.19, p <0.0001). The difference between vehicle-treated wild type (WT) and *Shank3b*^{*-*/*-*} knockout mice (KO) was significant (p <0.001). AMO-01 had no significant effect in wild type mice. In contrast, AMO-01 significantly reduced the deficit shown in *Shank3b*^{*-*/*-*} mice (p <0.0001) although this reversal was not complete since there was still a significant difference between AMO-01 treated wild type and *Shank3b*^{*-*/*-*} mice (p <0.05). "ns" is Not Significant; * is p < 0.05; **** is p < 0.0001.

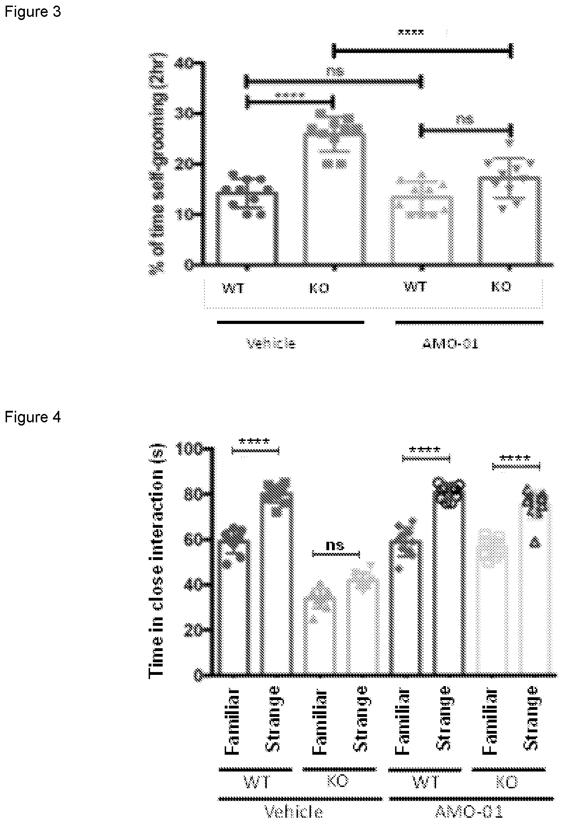

Figure 3. Self-injurious behavior as indexed by excessive self-grooming in *Shank3b* KO transgenic model of PMS after a single dose 30 mg/kg i.p. AMO-01. The overall ANOVA used to analyze this experiment was significant (F = 29.14, p <0.0001). The difference between vehicle-treated wild type and *Shank3b*^{*-*/*-*} knockout mice was significant (p <0.001). AMO-01 had no significant effect in wild type mice. In contrast, AMO-01 significantly reduced the deficit shown in *Shank3b*^{*-*/*-*} mice (p <0.0001). This reversal was complete since there was no significant difference between AMO-01 treated wild type and *Shank3b*^{*-*/*-*} mice after treatment. "ns" is Not Significant; **** is p < 0.0001
Figure 4. Response to social novelty as indexed by time spent in interaction with a familiar or novel mouse in *Shank3b* KO transgenic model of PMS after a single dose 30 mg/kg i.p. AMO-01. Wild Type animals (WT) spend more time in close interaction with a strange (not previously seen) than familiar mouse (one previously encountered) and this difference is not affected by treatment with AMO-01. This difference is not seen the *Shank3b* KO mice treated with vehicle showing disrupted processing of social novelty. Treatment with AMO-01 normalizes the differential response to social novelty in *Shank3b* KO mice. The overall ANOVA used to analyze this experiment was significant (F = 52.75, p <0.0001). "ns" is Not Significant; **** is p < 0.0001.

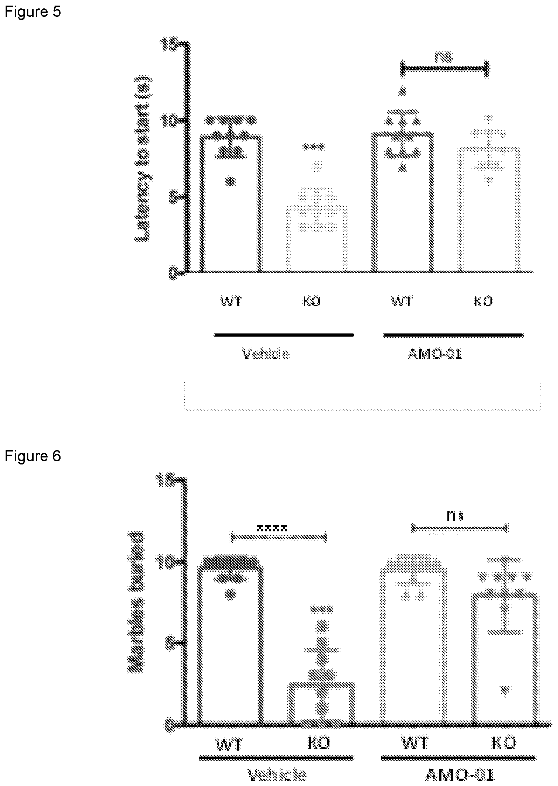

Figure 5. Beam walking behavior in *Shank3b* KO transgenic model of PMS after a single dose 30 mg/kg i.p. AMO-01. The overall ANOVA used to analyze this experiment was significant (F = 29.76, p <0.0001). The difference between vehicle treated wild type and *Shank3b*^{*-*/*-*} knockout mice was significant (p <0.001). AMO-01 had no significant effect in wild type mice. In contrast, AMO-01 significantly reduced the deficit shown in *Shank3b*^{*-*/*-*} mice (p <0.0001). This reversal was complete since there was no significant difference between AMO-01 treated wild type and *Shank3b*^{*-*/*-*} mice after treatment. "ns" is Not Significant; *** is p < 0.001.
Figure 6. Marble burying behavior in *Shank3b* KO transgenic model of PMS after a single dose 30 mg/kg i.p. AMO-01. The overall ANOVA used to analyze this experiment was significant (F = 42.15, p <0.0001). The difference between vehicle treated wild type and *Shank3b*^{*-*/*-*} knockout mice was significant (p <0.001). AMO-01 had no significant effect in wild type mice. In contrast, AMO-01 significantly reduced the deficit shown in *Shank3b*^{*-*/*-*} mice (p <0.0001). This reversal was complete since there was no significant difference between AMO-01 treated wild type and *Shank3b*^{*-*/*-*} mice after treatment. "ns" is Not Significant; *** is p < 0.001; **** is p < 0.0001.

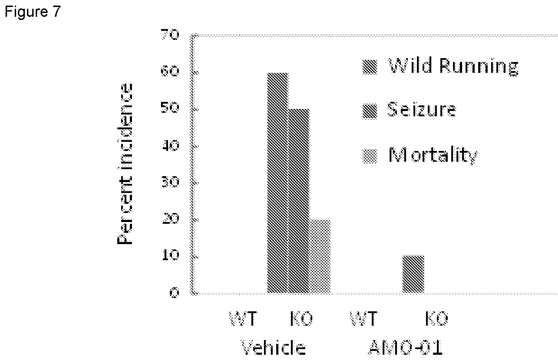

Figure 7. Audiogenic Seizure Threshold in *Shank3b* KO transgenic model of PMS after a single dose 30 mg/kg i.p. AMO-01.

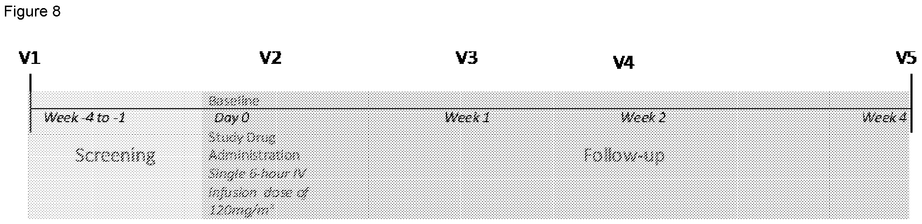

Figure 8. Schedule of administration of AMO-01 to Phelan McDermid Syndrome subj ect.

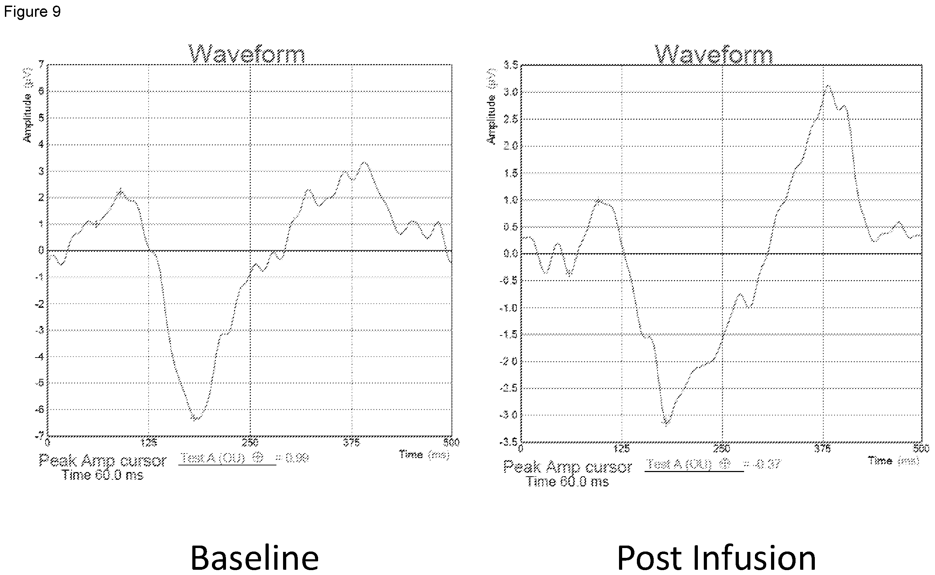

Figure 9. Visual Evoked Potentials recorded from visual cortex of a 23 year old male with genetically confirmed Phelan McDermid Syndrome immediately prior to and immediately after an infusion of a total dose administered as 120 mg/m² over six hours.

### DETAILED DESCRIPTION OF THE INVENTION

### I. Definitions

As used herein, "a" or "an" may mean one or more. As used herein when used in conjunction with the word "comprising," the words "a" or "an" may mean one or more than one. As used herein "another" may mean at least a second or more. Furthermore, unless otherwise required by context, singular terms include pluralities and plural terms include the singular.

As used herein, "about" refers to a numeric value, including, for example, whole numbers, fractions, and percentages, whether or not explicitly indicated. The term "about" generally refers to a range of numerical values (e.g., +/- 5-10% of the recited value) that one of ordinary skill in the art would consider equivalent to the recited value (e.g., having the same function or result). In some instances, the term "about" may include numerical values that are rounded to the nearest significant figure.

As used herein, "treat" and all its forms and tenses (including, for example, treat, treating, treated, and treatment) refer to both therapeutic treatment and prophylactic or preventative treatment. Subjects in need of treatment include those already diagnosed with PMS as well as those predisposed to develop PMS but not yet diagnosed as having PMS.

### II. The Present Invention

As indicated above, the present invention is based on the discovery by the inventors that certain farnesyl dibenzodiazepinone compounds can reduce symptoms of Phelan McDermid Syndrome (PMS) in individuals having the disorder and to whom the compounds are administered. Thus, the present invention is directed to compounds for use in methods for treating PMS in subjects having the disorder or in subjects predisposed to develop the disorder.

A subject having PMS or predisposed to develop PMS is a subject having a chromosomal deletion at 22q13.3 and/or in whom expression of SHANK3 protein is reduced and/or in whom a dysfunctional SHANK3 protein is produced. The chromosomal loci 22q13.3 includes the *SHANK3*/*ProSAP2* gene which encodes the SHANK3 protein.

SHANK3 ("SH3 and multiple ankyrin repeat domains 3"; also known as proline-rich synapse-associated protein 2 (ProSAP2)) is a scaffold protein found in dendrites. It is a member of a family of scaffold proteins present in post-synaptic densities involved in synapse formation, likely via connecting neurotransmitter receptors, ion channels, and other membrane proteins to the actin cytoskeleton and G-protein-coupled signaling pathways (Boeckers et al., 2002). SHANK3 message is expressed in heart, brain and spleen (Lim et al., 1999). Within the brain, SHANK3 is primarily expressed in neurons.

Studies have shown that different SHANK3 deletions produce differing (though similar) effects in terms of activity of SHANK3 protein isoforms and specific receptor changes. A variety of murine knockout mice in which the *Shank3* gene is curtailed have been created. Different exons of the gene have been addressed, with differing outcomes in relation to expression of isoforms of the SHANK3 protein. Bozdagi et al. (2010) created a mouse in which the *Shank3* gene was deleted between exons 4 and 9 (including ankyrin repeat domains). Heterozygous expression of this modification showed a 50% reduction *shank3* mRNA levels. In juvenile heterozygotes (3 to 4 weeks old) basal glutamatergic AMPA mediated transmission was reduced via pre- and post-synaptic mechanisms. Related to this, maintenance of high frequency and theta burst induced hippocampal Long Term Potentiation (LTP; a form of synaptic plasticity) was reduced, but Long Term Depression (LTD) was not. These electrophysiologically determined changes were paralleled by a failure to maintain the increased synapse volume that accompanies LTP, and reduced AMPA receptor trafficking. These mice showed social interaction deficits. Bozdagi et al. (2013) showed deficits in this model reversed by IGF-1.

Krouser et al. (2013) reported on homozygous deletion of *Shank3* exon 21 in mice studied at 2 to 6 months of age. This deletion reduced predominant isoforms of the SHANK3 protein in hippocampus but had complex effects with shorter isoforms appearing. This mouse showed no major changes in glutamate receptors except mGluR5 subtype of metabotropic glutamate receptor. Correspondingly, hippocampal LTP was deficient reflecting a reduced NMDA/AMPA ratio. There was no mGluR dependent LTD deficit in these mice. These mice showed no differences in dendritic spine density or complexity.

Duffney et al. (2015) created and reported on heterozygous mouse with c-terminal (exon 21) deletion of *Shank3* that showed a 50 to 70% reduction in SHANK3 protein. Juveniles showed impaired social interaction test behavior. NMDA mediated EPSPs in the pre-frontal cortex were reduced in these mice, whilst AMPA mediated responses were intact. This finding related to a reduction in membrane NMDA receptor subunits. These mice also showed a reduction in rac dependent cofilin signaling and therefore synaptic actin - however, dendritic spine density and therefore synapse number were not changed in these mice. The mice described by Bozdagi et al. (2010) had an N-terminus deletion leading to loss of the longest isoforms and also had cofilin deficiency. Uppal et al. (2015) studied the heterozygous N-terminus knockout at 5 weeks and 3 months of age and found no difference in synapse density at either age. However, detailed examination showed an increase in perforated synapses at 5 weeks but not 3 months. No differences at either age in spine, head size, length of the PSD, and area of the PSD were seen at any age. Jaramillo et al. (2016) reported on a heterozygous exon 4 to 9 N-terminus deletion in mice at 3 to 5 months of age at the time of study. This manipulation caused a complex pattern of changes in SHANK3 protein isoforms. These mice showed decreased hippocampal LTP with mGluR5 dependent LTD intact. Synaptic transmission and NDMA/AMPA ratio unaffected in hippocampus but NMDA/AMPA ratio decreased in striatum due to decreased NMDA receptor function.

Peca et al. (2011) produced a knockout mouse in which exons 13-16 of the *Shank3* gene were targeted, deleting the PDZ domain. This knockout produces a complete elimination of both SHANK3α (the longest isoform) and SHANKβ isoforms and a significant reduction of the putative SHANK3y isoform. The homozygous form of this mouse showed reduced levels of SAPAP3, Homer-1b/c and PSD93 as well as a reduction in the glutamate receptor subunits GluR2, NR2A and NR2B in the striatum. This was accompanied by a reduction in dendritic spine density, in the context of an increase in complexity of dendritic arbors and total dendritic length in the striatum. Striatal population spikes were reduced due to a reduction in post-synaptic AMPA responses in the absence of a change in NMDA/AMPA ratio. Hippocampal electrophysiology was not changed in these mice.

Therefore, in terms of synaptic effects, there is a broad loss of hippocampal synaptic plasticity across models, but different *Shank3* deletions produce differing effects in terms of SHANK3 protein isoforms and different specific receptor changes.

The effects of knockout of the *Shank3* gene in mice may be compared to IPS cell lines derived from Phelan McDermid Syndrome patients, as reported on by Shcheglovitov et al. (2013). These authors report that post-synaptic AMPA and NMDA responses are significantly impaired in Phelan McDermid Syndrome IPS cells, in the absence of differences in GABAergic inhibition. These changes reflected fewer excitatory synapses and are related to decreased levels of longest isoform of SHANK3 protein.

Holder and Quach (2016) cite the data of Shcheglovitov et al. (2013) in relation to the increased seizure susceptibility seen in Phelan McDermid Syndrome by referring to the IPCs studied as having increased input resistance compared to control neurons, potentially related to reduced expression of subunits of hyperpolarization-activated cyclic nucleotide-gated (HCN) channels. Holder and Quach propose this "indicates a tendency to increased excitability potentially underlying the elevated prevalence of epilepsy in individuals with SHANK3 mutations compared with the general population."

A key molecular aberration in PMS is over-activity of the Ras-ERK enzymatic pathway, which plays a critical role in synaptic plasticity and neuronal survival (Grewal et al., 1999). The Ras-ERK pathway has also been implicated in seizure generation according to a number of converging lines of evidence of activation of this pathway in different experimental models, and in the use of Ras-ERK pathway inhibitors. Ras-ERK pathway activation is indexed by levels of the phosphorylated form of ERK (pERK). This activation is seen during various forms of adaptation in synaptic mechanisms that underlie excitability in the brain. Davis et al., (2000) showed that induction of hippocampal LTP in the rat is accompanied by a rapid but transient increase in pERK.

On the basis of this over-activity, and as described below, a brain-penetrant inhibitor of the Ras-ERK pathway, termed AMO-01 herein, was assessed in a C57BL/6^{-/-} knockout mouse model of PMS. A single i.p. dose of AMO-01 administered to two-month old test animals was found to significantly improve all aberrant neurobehavioral features associated with the mice, thus establishing the basis for the present invention.

### Farnesyl Dibenzodiazepinones

AMO-01 (10-farnesyl-4,6,8-trihydroxy-dibenzodiazepin-11-one) is a farnesyl dibenzodiazepinone and a member of a class of dibenzodiazepinone compounds containing a farnesyl moiety. The structure of AMO-01 is as follows: 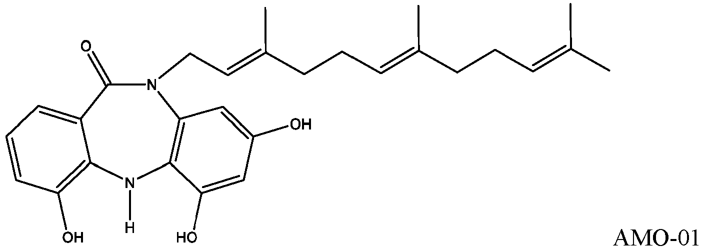 Each of the methods of the present invention may be practiced via administration of AMO-01, or a pharmaceutically acceptable salt thereof, to a subject having PMS or a subject predisposed to develop the PMS.

Farnesyl dibenzodiazepinone compounds are produced by strains of *Micromonospora,* a genus of bacteria of the family Micromonosporaceae that are gram-positive, spore-forming, generally aerobic, and that form a branched mycelium. Members of the genus also commonly produce aminoglycoside antibiotics.

AMO-01 is produced by *Micromonospora* sp. strain 046-ECO11. Strain 046-ECO11 was deposited on March 7, 2003, with the International Depositary Authority of Canada (IDAC), Bureau of Microbiology, Health Canada, 1015 Arlington Street, Winnipeg, Manitoba, Canada R3E 3R2, under Accession No. 070303-01. More details on strain 046-ECO11 and means for producing AMO-01 may be found in international patent publication WO 2004/065591, published August 5, 2004, the contents of which are incorporated herein by reference.

In addition to AMO-01, and pharmaceutically acceptable salts thereof, each of the methods of the present invention may be practiced via administration to a subject having PMS or a subject predisposed to develop PMS at least one compound of formula (I) or a pharmaceutically acceptable salt thereof: 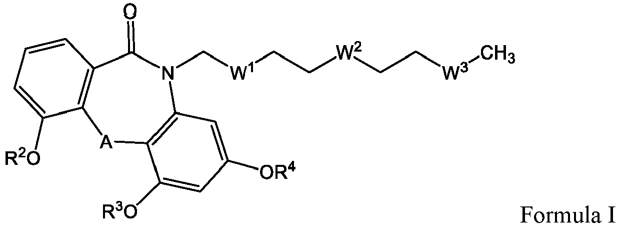 wherein,
each of W¹, W² and W³ is independently 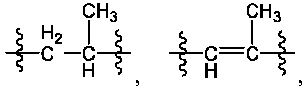 or the chain from the tricycle may terminate at W³, W² or W¹ with W³, W² or W¹ respectively being either -CH=O or -CH₂OH;
A is -NH-, or -NC(O)R¹-, where R¹ is C₁₋₆ alkyl,
each of R², R³, and R⁴ is independently H.

In certain embodiments, the invention provides compounds of Formula I, wherein A is selected from the group consisting of NH, and NC(O)R¹; wherein R² is H; R³ is H; and R⁴ is H. In another embodiment, R², R³ and R⁴ are each H; and all other groups are as previously defined. In a further embodiment, R², R³ and R⁴ are each H; and W¹ is -CH=CH- and all other groups are as previously defined. In a further embodiment, R², R³ and R⁴ are each H, and W² is -CH=CH- and all other groups are as previously defined. In a further embodiment, R², R³ and R⁴ are each H; and W³ is -CH=CH- ; and all other groups are as previously defined. In a further embodiment, A is NH; R², R³ and R⁴ are each H; and all other groups are as previously defined. In a further embodiment, A is NH; each of W¹, W², and W³ is -CH=CH-; and all other groups are as previously defined. The invention encompasses all pharmaceutically acceptable salts of the foregoing compounds.

The following are additional exemplary compounds of Formula I The compounds of formula IV, V, VI, XXVI, XXVII, XXVIII, XXIX, XXX, XXI, XXXII, XXXIII, XXXIV, XXXVII, XXXVIII and XXXIX are not of the invention. 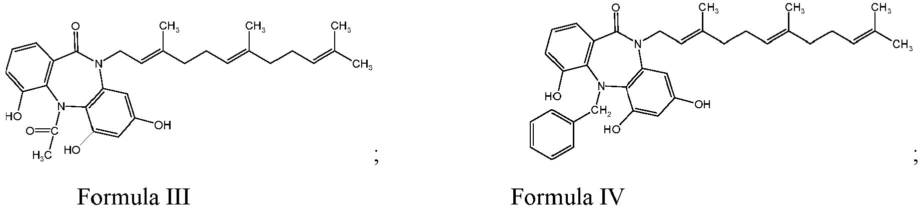 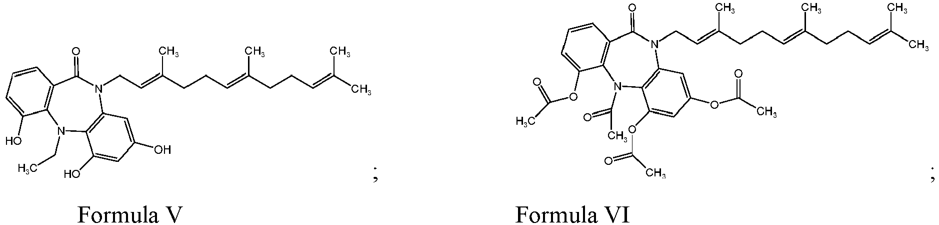 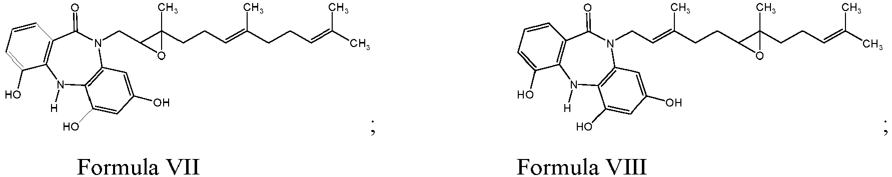 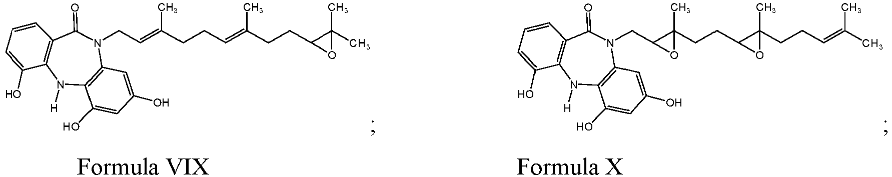 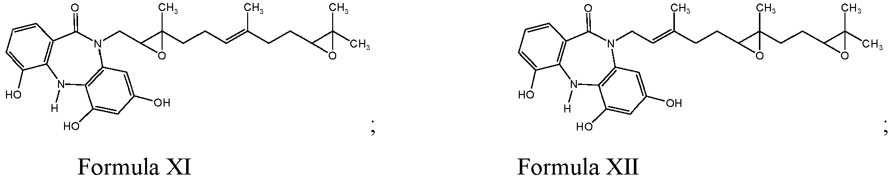 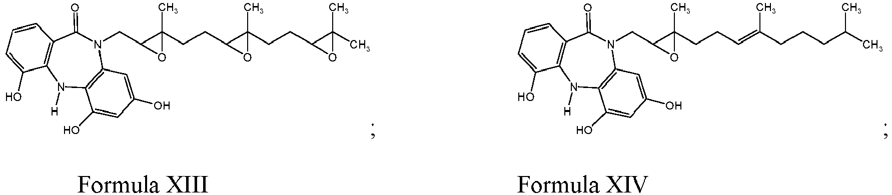 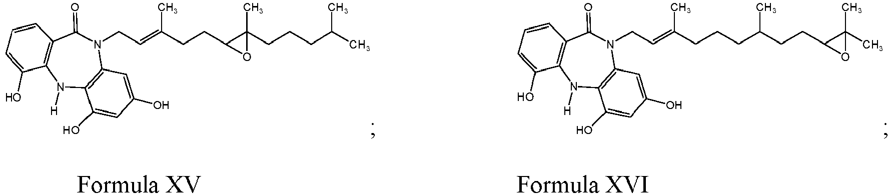 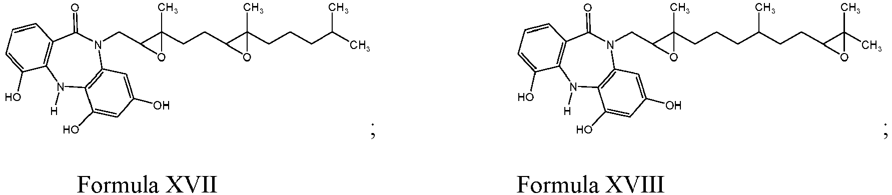 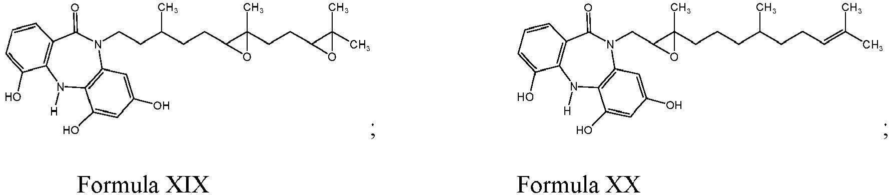 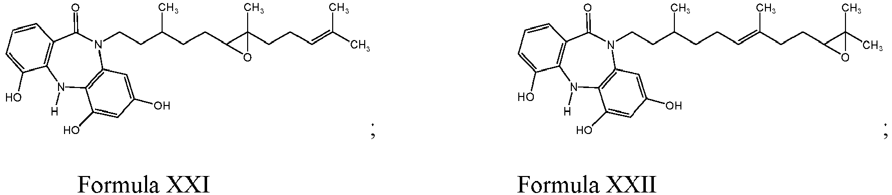 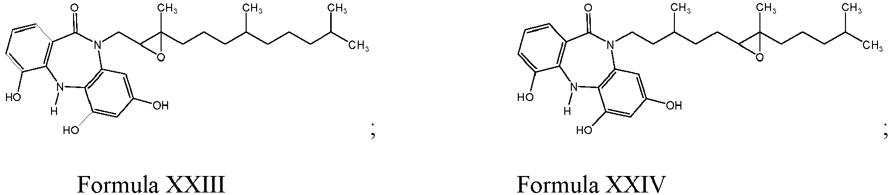 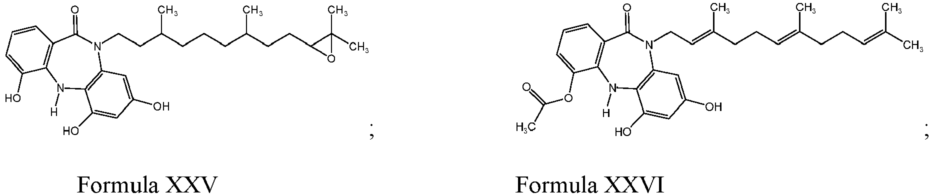 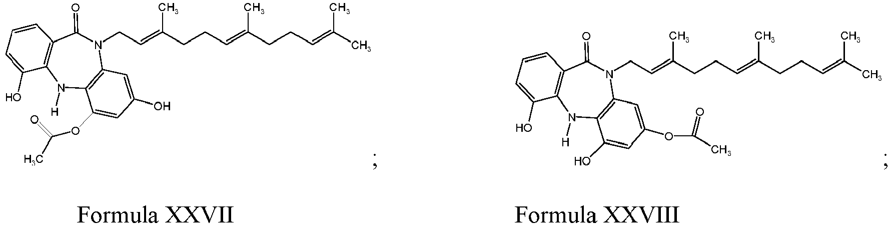 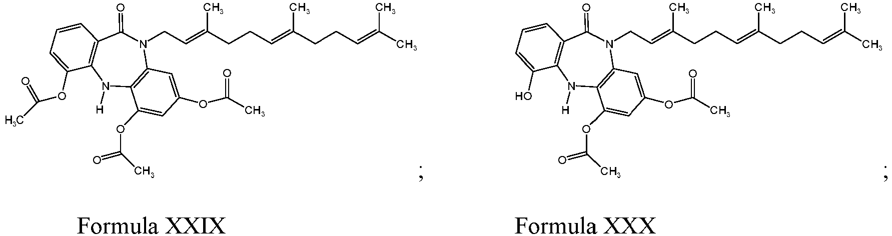 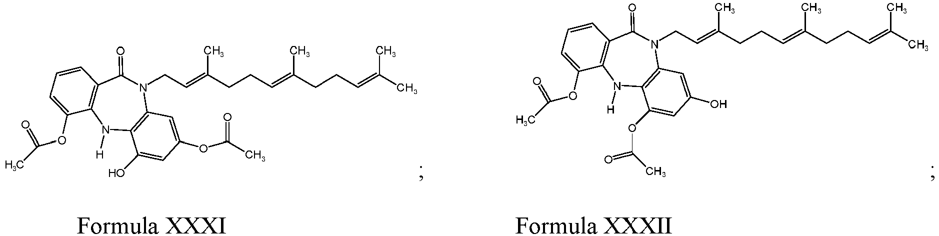 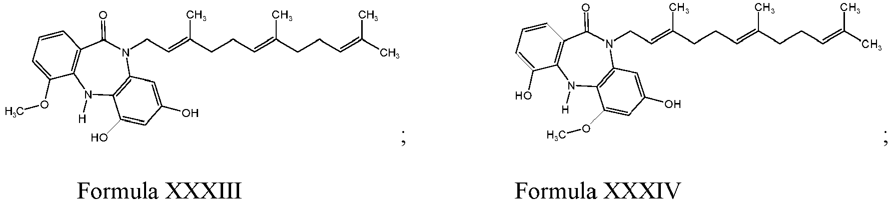 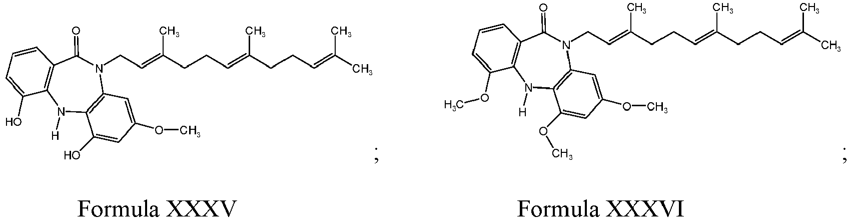 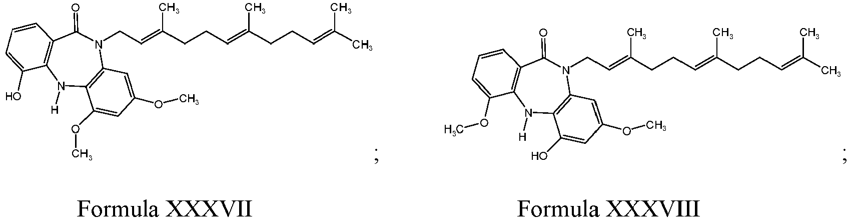 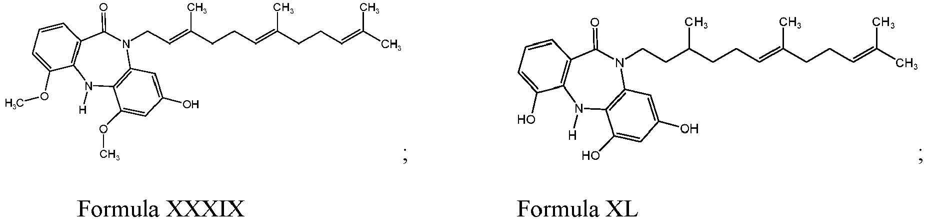 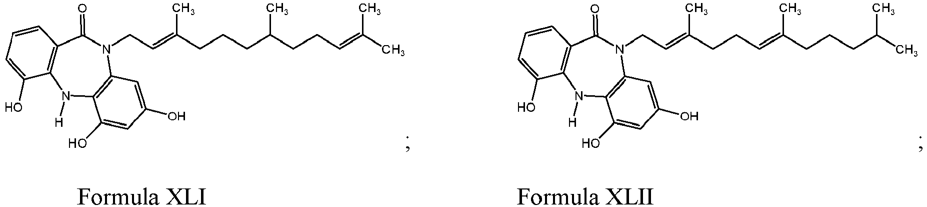 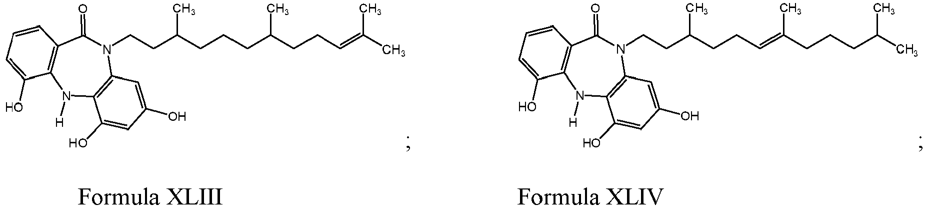 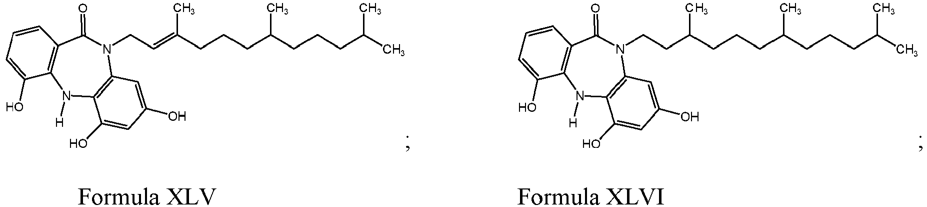 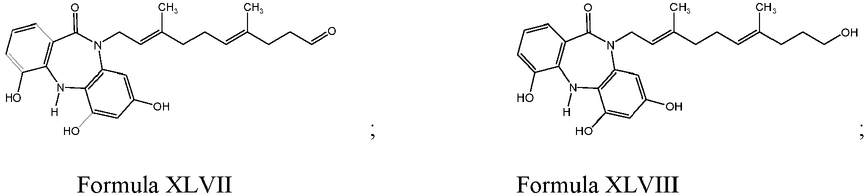 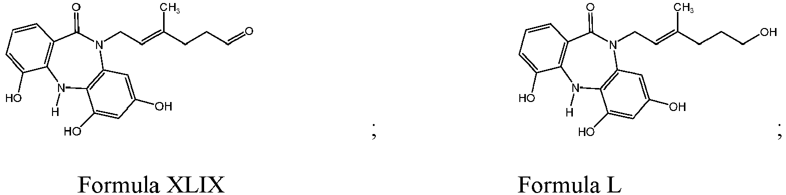 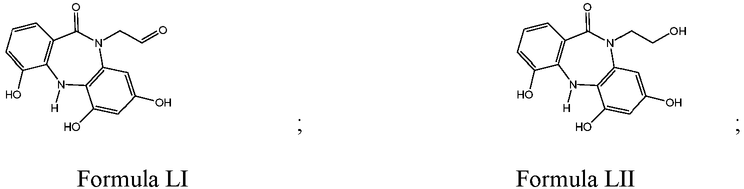 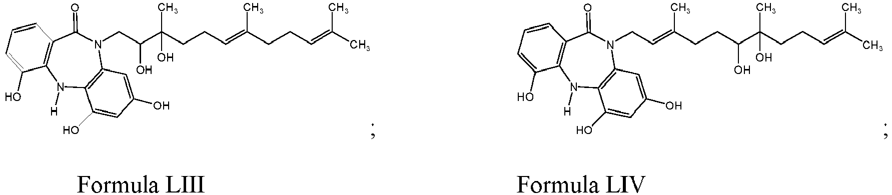 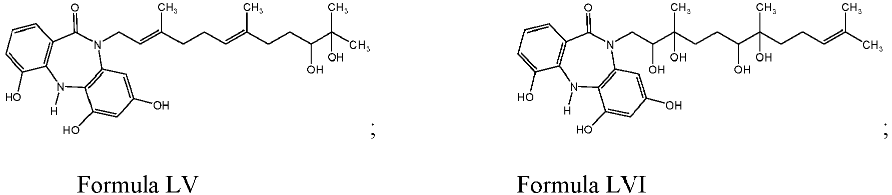 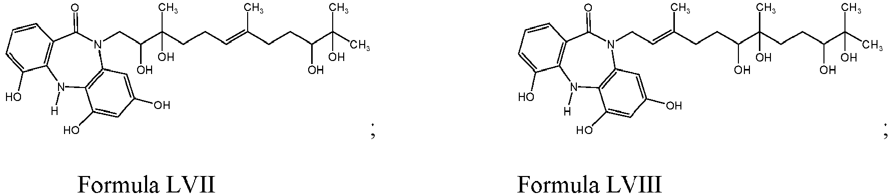 and

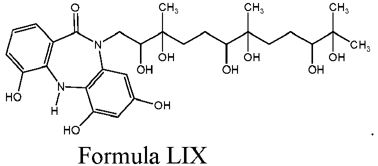

As used herein, the term "alkyl" refers to linear or branched hydrocarbon groups. Examples of alkyl groups include, without limitation, methyl, ethyl, n-propyl, isopropyl, n-butyl, pentyl, hexyl, heptyl, cyclopentyl, cyclohexyl, cyclohexymethyl, and the like. Alkyl may optionally be substituted with substituents selected from acyl, amino, acylamino, acyloxy, carboalkoxy, carboxy, carboxyamido, cyano, halo, hydroxyl, nitro, thio, alkyl, alkenyl, alkynyl, cycloalkyl, heterocyclyl, aryl, heteroaryl, alkoxy, aryloxy, sulfinyl, sulfonyl, oxo, guanidino and formyl.

The term "alkenyl" refers to linear, branched or cyclic hydrocarbon groups containing at least one carbon-carbon double bond. Examples of alkenyl groups include, without limitation, vinyl, 1-propen-2-yl, 1-buten-4-yl, 2-buten-4-yl, 1-penten-5-yl and the like. Alkenyl may optionally be substituted with substituents selected from acyl, amino, acylamino, acyloxy, carboalkoxy, carboxy, carboxyamido, cyano, halo, hydroxyl, nitro, thio, alkyl, alkenyl, alkynyl, cycloalkyl, heterocyclyl, aryl, heteroaryl, alkoxy, aryloxy, sulfinyl, sulfonyl, formyl, oxo and guanidino. The double bond portion(s) of the unsaturated hydrocarbon chain may be either in the cis or trans configuration.

The terms "cycloalkyl" and "cycloalkyl ring" refer to a saturated or partially unsaturated carbocyclic ring in a single or fused carbocyclic ring system having from three to fifteen ring members. Examples of cycloalkyl groups include, without limitation, cyclopropyl, cyclobutyl, cyclohexyl, and cycloheptyl. Cycloalkyl may optionally be substituted with substituents selected from acyl, amino, acylamino, acyloxy, carboalkoxy, carboxy, carboxyamido, cyano, halo, hydroxyl, nitro, thio, alkyl, alkenyl, alkynyl, cycloalkyl, heterocyclyl, aryl, heteroaryl, alkoxy, aryloxy, sulfinyl, sulfonyl and formyl.

The terms "heterocyclyl" and "heterocyclic" refer to a saturated or partially unsaturated ring containing one to four hetero atoms or hetero groups selected from O, N, NH, NRx, PO₂, S, SO or SO in a single or fused heterocyclic ring system having from three to fifteen ring members. Examples of a heterocyclyl or heterocyclic ring include, without limitation, morpholinyl, piperidinyl, and pyrrolidinyl. Heterocyclyl, heterocyclic or heterocyclyl ring may optionally be substituted with substituents selected from acyl, amino, acylamino, acyloxy, oxo, thiocarbonyl, imino, carboalkoxy, carboxy, carboxyamido, cyano, halo, hydroxyl, nitro, thio, alkyl, alkenyl, alkynyl, cycloalkyl, heterocyclyl, aryl, heteroaryl, alkoxy, aryloxy, sulfinyl, sulfonyl and formyl.

The term "amino acid" refers to any natural amino acid; natural amino acids are well known to a person skilled in the art.

The term "halo" refers to a halogen atom, e.g., bromine, chlorine, fluorine and iodine.

The terms "aryl" and "aryl ring" refer to aromatic groups in a single or fused ring system, having from five to fifteen ring members. Examples of aryl include, without limitation, phenyl, naphthyl, biphenyl, terphenyl. Aryl may optionally be substituted with one or more substituent group selected from acyl, amino, acylamino, acyloxy, azido, alkythio, carboalkoxy, carboxy, carboxyamido, cyano, halo, hydroxyl, nitro, thio, alkyl, alkenyl, alkynyl, cycloalkyl, heterocyclyl, aryl, heteroaryl, alkoxy, aryloxy, sulfinyl, sulfonyl and formyl.

The terms "heteroaryl" and "heteroaryl ring" refer to aromatic groups in a single or fused ring system, having from five to fifteen ring members and containing at least one hetero atom such as O, N, S, SO and SO₂. Examples of heteroaryl groups include, without limitation, pyridinyl, thiazolyl, thiadiazoyl, isoquinolinyl, pyrazolyl, oxazolyl, oxadiazoyl, triazolyl, and pyrrolyl groups. Heteroaryl groups may optionally be substituted with one or more substituent group selected from acyl, amino, acylamino, acyloxy, carboalkoxy, carboxy, carboxyamido, cyano, halo, hydroxyl, nitro, thio, thiocarbonyl, alkyl, alkenyl, alkynyl, cycloalkyl, heterocyclyl, aryl, heteroaryl, alkoxy, aryloxy, sulfinyl, sulfonyl, and formyl.

The terms "aralkyl" and "heteroaralkyl" refer to an aryl group or a heteroaryl group, respectively bonded directly through an alkyl group, such as benzyl. Aralkyl and heteroaralkyl may be optionally substituted as the aryl and heteroaryl groups.

Similarly, the terms "aralkenyl" and "heteroaralkenyl" refer to an aryl group or a heteroaryl group, respectively bonded directly through an alkene group, such as benzyl. Aralkenyl and heteroaralkenyl may be optionally substituted as the aryl and heteroaryl groups.

The compounds of the present invention can possess one or more asymmetric carbon atoms and can exist as optical isomers forming mixtures of racemic or non-racemic compounds. The compounds of the present invention are useful as single isomers or as a mixture of stereochemical isomeric forms. Diastereoisomers, i.e., nonsuperimposable stereochemical isomers, can be separated by conventional means such as chromatography, distillation, crystallization or sublimation. The optical isomers can be obtained by resolution of the racemic mixtures according to conventional processes.

As used herein, reference to a subject having PMS is a subject identified as having a chromosomal deletion at 22q13.3 having at least two of the following symptoms: hypotonia (low tone), normal or accelerated growth, absent or severely delayed speech, and global developmental delay.

As used herein, reference to a subject predisposed to develop PMS is a subject identified as having a chromosomal deletion at 22q13.3 and/or a subject having at least two of the following symptoms: hypotonia (low tone), normal or accelerated growth, absent or severely delayed speech, and global developmental delay.

As will be apparent, the identity of the chromosomal deletion(s) in a subject having PMS or predisposed to develop PMS can vary in size, number and specific location within the loci. Further, because PMS is thought to be related to haploinsufficiency of *SHANK3,* the chromosomal deletion at 22q13.3 may be in one or both of the alleles. When in both alleles, the deletion can be different between the alleles.

As used herein, reduced expression of SHANK3 protein in a subject is a reduction of SHANK3 protein expression of at least 30% versus a subject that does not have a chromosomal deletion at 22q13.3. The reduction in SHANK3 protein expression may be at least 30%, 35%, 40%, 45%, 50%, 55%, 60%, 65%, 70%, 75%, 80%, 85%, 90%, 95% or more versus a subject that does not have a chromosomal deletion at 22q13.3.

As used herein, a dysfunctional SHANK3 protein is a SHANK3 protein that does not support normal synapse function. *III.* Compounds for use in methods of treatment

The present invention is drawn to compounds for use in methods of treating a subject having PMS or a subject predisposed to develop PMS. Each of the methods of the invention comprise administering a therapeutically effective amount of (i) at least one compound of formula (I) or (ii) a pharmaceutical formulation comprising at least one compound of formula (I) and a pharmaceutically acceptable carrier or diluent to a subject, such as a subject having PMS or a subject predisposed to develop PMS. In certain embodiments, the at least one farnesyl dibenzodiazepinone compound is AMO-01.

Also disclosed is the use of (i) at least one farnesyl dibenzodiazepinone compound or (ii) a pharmaceutical formulation comprising at least one farnesyl dibenzodiazepinone compound and a pharmaceutically acceptable carrier or diluent in (a) treating a subject having PMS, (b) methods of treating a subject having PMS, (c) treating a subject predisposed to develop PMS or (d) methods of treating a subject predisposed to develop PMS. In certain embodiments, the at least one farnesyl dibenzodiazepinone compound is AMO-01.

Also disclosed is a therapeutically effective amount of (i) at least one farnesyl dibenzodiazepinone compound or (ii) a pharmaceutical formulation comprising at least one farnesyl dibenzodiazepinone compound and a pharmaceutically acceptable carrier or diluent for use in (a) treating a subject having PMS, (b) a method of treating a subject having PMS, (c) treating a subject predisposed to develop PMS, or (d) methods of treating a subject predisposed to develop PMS. In certain embodiments, the at least one farnesyl dibenzodiazepinone compound is AMO-01.

As used herein, the terms "treat", "treating" and "treatment" have their ordinary and customary meanings, and include one or more of the following: ameliorating a symptom of PMS in a subject having PMS or a subject predisposed to develop PMS; blocking a symptom of PMS in such a subject; blocking or ameliorating recurrence of a symptom of PMS in such a subject; decreasing in severity and/or frequency a symptom of PMS in such a subject; blocking progression of PMS in such a subject; and curing or resolving PMS in such a subject. Treatment means ameliorating, etc. by about 1% to about 100% versus a subject to whom the treatment has not been administered. Preferably, the ameliorating, etc. is about 100%, about 99%, about 98%, about 97%, about 96%, about 95%, about 90%, about 80%, about 70%, about 60%, about 50%, about 40% or about 30% versus a subject to whom the treatment has not been administered. The treatment may begin prior to, concurrent with, or after the onset of clinical symptoms of the disorder. Thus, the subject may be showing symptoms of PMS, or merely diagnosed as predisposed to develop PMS but so far free of symptoms of the disorder. The results of the treatment may be permanent or may continue for a period of days (such as 1, 2, 3, 4, 5, 6 or 7 days), weeks (such as 1, 2, 3 or 4 weeks) or months (such as 1, 2, 3, 4, 5, 6 or more months).

The amount of the at least one farnesyl dibenzodiazepinone administered to a subject when the methods of the invention are practiced will vary by such factors as the age and weight of the subject, and the identity and severity of the symptoms of the disorder. However, the amount administered to a subject will be a therapeutically effective amount, i.e. an amount sufficient to effect treatment. As an example, a therapeutically-effective amount of the at least one farnesyl dibenzodiazepinone is between about 0.1 ug to about 200 mg per kg of body weight of the subject. Additional ranges of therapeutically-effective amounts include, but are not limited to, about 10 ug/kg to 200 mg/kg; about 100 ug/kg to 200 mg/kg; about 1 mg/kg to 200 mg/kg; about 10 mg/kg to 200 mg/kg; about 10 ug/kg to 100 mg/kg; about 100 ug/kg to 100 mg/kg; about 1 mg/kg to 100 mg/kg; about 10 mg/kg to 100 mg/kg; about 0.1 ug/kg to 50 mg/kg; about 1 ug/kg to 50 mg/kg; about 10 ug/kg to 50 mg/kg; about 100 ug/kg to 50 mg/kg; about 1 mg/kg to 50 mg/kg; about 1 mg/kg to 40 mg/kg; about 1 mg/kg to 30 mg/kg; about 1 mg/kg to 20 mg/kg; about 1 mg/kg to 10 mg/kg; about 1 mg/kg to 5 mg/kg; about 0.1 mg/kg to 10 mg/kg; about 0.1 mg/kg to 5 mg/kg; about 10 mg/kg to 50 mg/kg; and about 20 ug/kg to 40 mg/kg of body weight of the subject. Specific examples of therapeutically-effective amounts include, but are not limited to, about 0.1, 0.5, 1, 1.5, 2, 2.5, 3, 3.5, 4, 4.5, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 and 60 mg/kg of body weight of the subject. Stated in an alternative manner, a therapeutically-effective amount of the at least one farnesyl dibenzodiazepinone is between about 1 mg/m² to about 1000 mg/m². Additional ranges of therapeutically-effective amounts include, but are not limited to, about 1 mg/m² to about 1000 mg/m²; about 1 mg/m² to about 500 mg/m²; about 1 mg/m² to about 400 mg/m²; about 1 mg/m² to about 300 mg/m²; about 1 mg/m² to about 250 mg/m²; about 1 mg/m² to about 200 mg/m²; about 1 mg/m² to about 150 mg/m²; about 1 mg/m² to about 100 mg/m²; about 50 mg/m² to about 1000 mg/m²; about 50 mg/m² to about 500 mg/m²; about 50 mg/m² to about 400 mg/m²; about 50 mg/m² to about 300 mg/m²; about 50 mg/m² to about 200 mg/m²; about 50 mg/m² to about 150 mg/m²; about 50 mg/m² to about 100 mg/m²; about 100 mg/m² to about 1000 mg/m²; about 100 mg/m² to about 500 mg/m²; about 100 mg/m² to about 400 mg/m²; about 100 mg/m² to about 300 mg/m²; about 100 mg/m² to about 200 mg/m²; and about 100 mg/m² to about 150 mg/m². Specific examples of therapeutically-effective amounts include, but are not limited to, about 10, 20, 30, 40, 50, 60, 70, 80, 90, 100, 110, 120, 130, 140, 150, 160, 170, 180, 190, or 200 mg/m², or more.

The at least one farnesyl dibenzodiazepinone compound may be administered to a subject once, or twice, three times, four times, five times, six times or more, over a course of treatment. The timing between each dose in a dosing schedule may range between days, weeks, months, or years, an includes administered once every 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or more weeks. The same quantity of the at least one farnesyl dibenzodiazepinone may be administered in each dose of the dosing schedule, or the amounts in each dose may vary. Appropriate doses and dosing schedules can be readily determined by techniques well known to those of ordinary skill in the art without undue experimentation. Such a determination will be based, in part, on the tolerability and efficacy of a particular dose.

Administration frequencies include 4, 3, 2 or once daily, every other day, every third day, every fourth day, every fifth day, every sixth day, once weekly, every eight days, every nine days, every ten days, bi-weekly, monthly and bi-monthly. The duration of treatment will be based on the symptoms and severity of the disorder in the particular subject and will be best determined by the attending physician. However, continuation of treatment is contemplated to last for a number of days, weeks, months or years. Indeed, in some instances, treatment may continue for the entire life of the subject.

Depending on the means of administration, the dosage may be administered all at once, such as with an oral formulation in a capsule or liquid, or slowly over a period of time, such as with an intramuscular or intravenous administration. When administered over time, the course of administration may be, for example, 10, 20, 30, 40, 50 or 60 minutes, or 0.5, 1, 1.5, 2, 2.5, 3, 3.5, 4, 4.5, 5, 5.5, 6, 6.5, 7, 7.5, 8, 8.5, 9, 9.5, or 10 hours, or more. In one embodiment of the invention, the dose is 120 mg/m² delivered to the subject over 6 hours.

As used herein, "subject" includes, but is not limited, to a human, a non-human primate, bird, horse, cow, goat, sheep, a companion animal, such as a dog, cat or rodent, or other mammal.

### IV. Formulations and Doses

In each of the methods of treatment of the invention, the at least one compound of formula (I) and pharmaceutical formulations are administered in a pharmaceutically acceptable form and in substantially non-toxic quantities.

The pharmaceutical formulations of the invention will comprise at least one farnesyl dibenzodiazepinone compound as described herein. The pharmaceutical formulations will also comprise one or more pharmaceutically acceptable carrier or diluent.

Suitable examples of carriers and diluents are well known to those skilled in the art and include water, water-for-injection, saline, buffered saline, dextrose, glycerol, ethanol, propylene glycol, polysorbate 80 (Tween-80^{™}), poly(ethylene)glycol 300 and 400 (PEG 300 and 400), PEGylated castor oil (e.g. Cremophor EL), poloxamer 407 and 188, hydrophilic and hydrophobic carriers, and combinations thereof. Hydrophobic carriers include, for example, fat emulsions, lipids, PEGylated phospholipids, polymer matrices, biocompatible polymers, lipospheres, vesicles, particles, and liposomes. The terms specifically exclude cell culture medium. The formulations may further comprise stabilizing agents, buffers, antioxidants and preservatives, tonicity agents, bulking agents, emulsifiers, suspending or viscosity agents, inert diluents, fillers, and combinations thereof.

The identity of the carriers and diluents will depend on the manner in which the pharmaceutical formulations are being used and the means used to administer the pharmaceutical formulations to a subject. For example, pharmaceutical formulations for intramuscular preparations can be prepared where the carrier is water-for-injection, 0.9% saline, or 5% glucose solution.

Additionally, the pharmaceutical formulations may be administered in a liquid form. The liquid can be for oral dosage, for ophthalmic or nasal dosage as drops, or for use as an enema or douche. When the pharmaceutical formulation is formulated as a liquid, the liquid can be either a solution or a suspension of the pharmaceutical formulation. There is a variety of suitable formulations for the solution or suspension of the pharmaceutical formulations that are well known to those of skill in the art, depending on the intended use thereof. Liquid formulations for oral administration prepared in water or other aqueous vehicles may contain various suspending agents such as methylcellulose, alginates, tragacanth, pectin, kelgin, carrageenan, acacia, polyvinylpyrrolidone, and polyvinyl alcohol. The liquid formulations may also include solutions, emulsions, syrups and elixirs containing, together with the active compound(s), wetting agents, sweeteners, and coloring and flavoring agents.

The farnesyl dibenzodiazepinone compounds and pharmaceutical formulations may be formulated for and administered via any of the means commonly known, including, but not limited to oral, sublingual, intranasal, intraocular, rectal, transdermal, mucosal, pulmonary, topical or parenteral administration. Parenteral modes of administration include without limitation, intradermal, subcutaneous (s.c., s.q., sub-Q, Hypo), intramuscular (i.m.), intravenous (i.v.), intraperitoneal (i.p.), intra-arterial, intramedulary, intracardiac, intra-articular (joint), intrasynovial (joint fluid area), intracranial, intraspinal, and intrathecal (spinal fluids). Any known device useful for parenteral injection or infusion of drug formulations can be used to effect such administration.

### V. Example 1

### Mice

*Shank3b*^{*-*/*-*} homozygous mice with deletions of exons 13-16, initially obtained from the Jackson Laboratory, and wild type (WT) littermates were generated on a C57BL/6J background and repeatedly backcrossed onto a C57BL/6J background for more than eight generations. The resulting *Shank3* knockout (KO) mice were housed in groups of the same genotype in a temperature and humidity controlled room with a 12-h light-dark cycle (lights on 7 am to 7 pm). Room temperature and humidity were recorded continuously in the holding room. Testing was conducted during the light phase. Food and water were available *ad libitum.* Testing was conducted on *Shank3* knockout mice and their wild type littermates, with N = 10 mice per treatment group, tested at two months of age during behavioral experiments. Mice were housed in commercial plastic cages and experiments were conducted in line with the requirements of the UK Animals (Scientific Procedures) Act, 1986. All experiments were conducted with experimenters blind to genotype and drug treatment. Animals were allowed a minimum acclimatization period of one week prior to performing any experiment. Only healthy animals were used. No prophylactic or therapeutic treatment was to be administered during the acclimatization period.

### Ras-ERK activity

In an initial experiment, activity of the Ras-ERK pathway in the brain and lymphocytes was determined in treatment versus naive animals. Groups of N = 10 mice were compared. For determination of brain biomarker levels, the following methods and reagents were used. Twenty micrograms of protein lysate from each sample was separated on 8-16% sodium dodecyl sulfate polyacrylamide electrophoresis gels (Bio-Rad, 161-1222) and then transferred to nitrocellulose membranes (Bio-Rad, 162-0115). Membranes were processed following the ECL western blotting substrate (Pierce, 32106) with (ERK) 1/2 (1/2000) (Cell Signaling). Total ERK 1/2 protein content (phosphorylated) was evaluated by blotting membranes with antiphospho-ERK antibodies (1/2000) (Cell Signaling). ERK phosphorylation was normalized to protein content in the same sample and expressed as % of change with respect to basal conditions, considering basal levels as 100%. Protein loading was evaluated by stripping and reblotting membranes with b-actin antibody (1/1000) (Sigma Chemical Co.). Phosphor-ERK1/2 (Thr-202/Tyr-204) (Cell Signaling Technology^{®}, #4370) total ERK, p44/42 MAPK (Erk1/2) Antibody #9102 (Cell Signaling Technology^{®}). For loading controls, membranes were stripped and re-probed with the antibody against tubulin (Sigma, A3 853). For lymphocyte biomarker determinations, a FACStar plus (Becton Dickinson) was used with the excitation laser tuned at 488 nm and green fluorescence from FITC (GST) was collected through a 515-545 nm bandpass filter. The mean FITC fluorescence intensity was calculated in relation to the fluorescence of reference cells. The mean cellular fluorescence intensity (MFI) is directly proportional to the mean number of Ab molecules bound per cell.

### Behavioral Testing

AMO-01 from AMO Pharma Ltd was administered to mice as follows:
A. AMO-01 at a single dose of 30 mg/kg intraperitoneally in a vehicle of ethanol, PEG 400 and polysorbate 80 and 5% dextrose
B. Vehicle dosed intraperitoneally as 15 ml/kg
Groups of N = 10 mice were treated 4 hours before testing on the first day of testing. Five days of testing were undertaken following the single intraperitoneal dose administered on Day 1.

The following groups were studied:
1. *Shank3b*^{*-*/*-*} knockout mice + vehicle, N = 10
2. *Shank3b*^{*-*/*-*} knockout mice + AMO-01 30 mg/kg i.p., N = 10
3. Wild Type litter mates + vehicle, N = 10
4. Wild Type litter mates + AMO-01 30 mg/kg i.p., N = 10

The following tests were administered.

Day 1: Increased anxiety in the light dark box test. Testing was conducted in a two-chambered test apparatus (Med Associates, St. Albans, VT), with one side "the dark-chamber" kept in the dark at all times and the other chamber illuminated through testing was "the light-chamber". Upon placing the mice into the dark chamber, the light chamber was illuminated and the door between the two chambers was opened. The mice were allowed to freely explore the apparatus for 5 min. The latency to emerge from the darkened into the lighted chamber and the percentage of time spent in the illuminated chamber were used as indices of anxiety-like behaviours.

Day 2: Excessive grooming leading to skin lesions. *Shank3b*^{*-*/*-*} knockout mice develop pronounced skin lesions in varying degrees in the general holding colony. During this test, time spent grooming in all sequences: face-wiping, scratching/rubbing of head and ears, and full-body grooming was scored by an observer blind to genotype during the scoring.

Day 3: Abnormalities in social recognition and response to social novelty. This test was composed of three phases with various stimuli placed in each of two side chambers. Phase 1 contains two identical nonsocial stimuli (NS1 & NS1), phase 2 contains a nonsocial stimulus (NS1) and a social stimulus (Sod), and phase 3 contains a known social stimulus (Soc1) and a novel social stimulus (Soc2). The preference index for one stimulus over the other stimulus in each phase was compared.

Day 4: Sensory motor function as assessed by beam walking tests. A standard paradigm to assess beam walking performance (latency to fall) was used.

Day 5: Activities of daily living / species typical behaviors as assessed by marble burying. A standard paradigm was used to assess marble burying behavior.

Seizure threshold as assessed by audiogenic seizures was assessed in a separate cohort with N = 10 per group. Seizures were induced with a 60s continuous stimulus (124 db). A blinded observer rated motor response rates and Seizure Severity Score after auditory stimulation according to the classification: wild running, seizure and respiratory arrest.

Results from Ras-ERK pathway function testing are shown in Figure 1 where activation of the Ras-ERK pathway in Wild Type (WT) and *shank3* knockout (KO) mice as indexed by pERK levels in hippocampus and cortex as shown. No changes were seen in the hippocampus, which parallels the lack of effect seen in this region using electrophysiology in the studies of Peca et al. (2011). Conversely, an almost three fold increase was seen in pERK in the cortex (Peca et al. 2011 reported striato-cortical changes in this mouse). A significant increase was also seen in peripherally circulating lymphocytes (data not shown).

Results from the light dark box test are provided in Figure 2 which shows rescue of light dark box anxiety mediated behaviors by AMO-01 in *Shank3b* KO mice. Anxiety as assessed by light dark box testing in *Shank3b* KO transgenic model of PMS after a single dose 30 mg/kg ip AMO-01 or vehicle administered 4 hours before testing in groups of N = 10 mice. The overall ANOVA used to analyse this experiment was significant (F = 31.19, p <0.0001). The difference between vehicle treated Wild Type and *Shank3b*^{*-*/*-*} knockout (KO) mice was significant (p <0.001). AMO-01 had no significant effect in Wild Type mice. AMO-01 significantly reduced the deficit shown in *Shank3b*^{*-*/*-*} mice (p <0.0001) although this reversal was not complete since there was still a significant difference between AMO-01 treated Wild Type and *Shank3b*^{*-*/*-*} KO mice (p <0.05).

Results from the grooming test are provided in Figure 3 which shows a rescue of excessive grooming behavior by AMO-01 in *Shank3b* KO mice. Self-injurious behavior as indexed by excessive self-grooming in *Shank3b* KO transgenic model of PMS after a single dose 30 mg/kg ip AMO-01. The overall ANOVA used to analyse this experiment was significant (F = 29.14, p <0.0001). The difference between vehicle treated Wild Type and *Shank3b*^{*-*/*-*} knockout mice was significant (p <0.001). AMO-01 had no significant effect in Wild Type mice. AMO-01 significantly reduced the deficit shown in *Shank3b*^{*-*/*-*} mice (p <0.0001). This reversal was complete since there was no significant difference between AMO-01 treated Wild Type and *Shank3b*^{*-*/*-*} KO mice after treatment.

Results from the social recognition and response to social novelty testing are provided in Figure 4 which shows a rescue of response to social novelty by AMO-01 in *Shank3b* KO mice. Response to social novelty was indexed by time spent in interaction with a familiar or novel mouse in *Shank3b* KO transgenic model of PMS after a single dose 30 mg/kg ip AMO-01 The overall ANOVA used to analyse this experiment was significant (F = 52.75, p <0.0001).

Results from the beam walking tests are provided in Figure 5 which shows a rescue of beam walking behavior by AMO-01 in *Shank3b* KO mice. Beam walking behavior in *Shank3b* KO transgenic model of PMS after a single dose 30 mg/kg ip AMO-01. The overall ANOVA used to analyse this experiment was significant (F = 29.76, p <0.0001). The difference between vehicle treated Wild Type and *Shank3b*^{*-*/*-*} knockout mice was significant (p <0.001). AMO-01 had no significant effect in Wild Type mice. AMO-01 significantly reduced the deficit shown in *Shank3b*^{*-*/*-*} mice (p <0.0001). This reversal was complete since there was no significant difference between AMO-01 treated Wild Type and *Shank3b*^{*-*/*-*} KO mice after treatment.

Results from the marble burying test are provided in Figure 6 which shows a rescue of marble burying behavior by AMO-01 in *Shank3b* KO mice. Marble burying behavior in *Shank3b* KO transgenic model of PMS after a single dose 30 mg/kg ip AMO-01. The overall ANOVA used to analyse this experiment was significant (F = 42.15, p <0.0001). The difference between vehicle treated Wild Type and *Shank3b*^{*-*/*-*} knockout mice was significant (p <0.001). AMO-01 had no significant effect in Wild Type mice. AMO-01 significantly reduced the deficit shown in *Shank3b*^{*-*/*-*} KO mice (p <0.0001). This reversal was complete since there was no significant difference between AMO-01 treated Wild Type and *Shank3b*^{*-*/*-*} KO mice after treatment.

Results from the seizure threshold test are provided in Figure 7 which shows a rescue of lowered audiogenic seizure threshold by AMO-01 in *Shank3b* KO mice. Audiogenic Seizure Threshold in *Shank3b* KO transgenic model of PMS after a single dose 30 mg/kg ip AMO-01.

It can thus be seen that the experiments provided herein showed that in the *Shank3b*^{*-*/*-*} homozygous knockout mouse model of Phelan McDermid Syndrome the Ras-ERK pathway is activated in the cortex (by a significant multiple) whilst activity in unchanged in the hippocampus. These data are consistent with those reported by Peca et al. (2011) who showed altered plasticity of striato-cortical pathways in the absence of hippocampal changes in this model. An increase of Ras-ERK pathway activation is consistent with a lowering of seizure threshold in the cortex Nateri et al. (2007).

Administration of 30 mg/kg i.p. AMO-01 was without effect on anxiety, grooming, sociability, sensori-motor function or species typical behaviours or cognition in Wild Type mice. These data are consistent with previous behavioral testing results in Wild Type mice with this compound. In contrast, this dose reversed deficits in all of these domains in the *Shank3* knockout transgenic mouse model of Phelan McDermid Syndrome. This reversal represented a significant normalization of function in the anxiety and audiogenic seizure domains, and a complete normalization of function in the grooming, social, sensori-motor and species typical. This broad rescue of the Phelan McDermid Syndrome phenotype was engendered after a single dose of AMO-01, and persisted for at least five days.

### VI. Example 2

A twenty-three year old male with a genetically confirmed diagnosis of Phelan McDermid Syndrome was administered AMO-01.

The study drug was prepared from a sterile bulk formulation containing 32.8 mg/mL (3.15% w/w) of the active drug substance dissolved in sterile 0.9% saline solution to produce an isotonic final dosing formulation. Each drug product vial contained 70 mg of AMO-01 drug substance in 2.134 mL of formulation (plus 2% overage). This corresponded to a total drug content of 71.4 mg of active drug substance per 2.176 mL of formula. AMO-01 was administered as a single dose via intravenous infusions over a 6 hour period, with the total dose administered as 120 mg/m².

Seizure frequency and Visual Evoked Potentials (VEPs) were assessed alongside clinical measures. VEPs provide a noninvasive technique to evaluate the functional integrity of visual pathways in the brain from the retina to the visual cortex via the optic nerve/optic radiations. The VEP is recorded from the head's surface, over the visual cortex, and is extracted from ongoing EEG through signal averaging. VEPs reflect the sum of excitatory and inhibitory postsynaptic potentials occurring on apical dendrites (Zemon et al., 1986) which modulate excitatory and inhibitory signals received by the pyramidal cells. The major positive and negative peaks and troughs in VEP waveforms reflect different cellular events. The contrast-reversing checkerboard stimulus used in this study produces a positive peak at approximately 60 ms (P0 or P60), reflecting activation of the primary visual cortex from the lateral geniculate nucleus. A negative peak at approximately 75 ms (N0 or N75) reflecting depolarization and glutamatergic postsynaptic activity spreading to the superficial layers of primary visual cortex, and a positive peak at approximately 100 ms (P1 or P100) reflecting superficial hyperpolarization and GABAergic activity (Zemon et al., 1980). VEPs have been used in clinical trials; for example, an antiepileptic drug (gabapentin; Conte et al., 2009) and an infant formula (O'Connor et al., 2009). Both received FDA approval based in part on positive findings from VEP studies. Data from ongoing clinical trials at Mount Sinai provide support for use of VEPs as a measure of treatment response. Assessments of clinical response were made before treatment, after six hours of treatment, and one week, two weeks and four weeks after treatment according to the schematic in Figure 8.

### Results

The subject had a well-documented history of mixed seizures that had been occurring "like clockwork" 3 to 4 times a week, every week, for four years since he was nineteen years of age. His seizures included tonic-clonic convulsions accompanied by oxygen desaturation, as well as orthostatic blood pressure changes. These seizures were of a severe, serious nature and were potentially life-threatening events.

The subject was on the threshold of having one of these severe seizures in the hour or so preceding the study drug infusion - his pre-seizure peri-ictal period being detectable due to being accompanied by classic personality and behavioral changes. According to investigating clinician, the infused study drug appeared to completely abort the impending seizure episode. Remarkably, the seizures stopped altogether for the next two weeks (and according to investigating clinician, this was the first run of two weeks without seizures since the subject was in his mid- to late-teens, at least five years ago. During this seizure-free period, this young man "awakened" on several different levels and he became more social, more engaged (e.g. better eye contact), more cooperative, less restless, and his motor skills also improved. Approximately three weeks after the study drug infusion, "little events" began to recur, perhaps representing minor complex partial seizures, albeit not accompanied by convulsions - these rare/sporadic episodes were described by the investigating clinician as "briefer, with less movement involved".

VEPs were recorded immediately before and six hours after the infusion of AM0-01. Figure 9 shows how the VEPs from this subject were ameliorated by treatment.

Collectively, these data confirm that administration of AMO-01 to a human subject with loss of function of the SHANK3 gene can result in clinical benefit across multiple features of Phelan McDermid Syndrome that persist beyond exposure to this compound, as is seen *in vivo* in the Shank3b knockout mouse model of this disorder.

### REFERENCES

All of the following references have been cited in this application:
Anderlid BM, Schoumaus J, Anneren G, Paez I, Dumanski J, Blennow E, Nordenskjöld M. (2002) FISH-mapping of a 100-kb terminal 22q 13 deletion. Hum Genet. 110:439-443.
Boeckers TM, Bockmann J, Kreutz MR, Gundelfinger ED. (2002) ProSAP/Shank proteins - a family of higher order organizing molecules of the postsynaptic density with an emerging role in human neurological disease. J Neurochem. 81:903-910.
Bonaglia M, Giorda R, Borgatti R, Felisari G, Gagliardi C, Selicorni A, Zuffardi O. (2001) Disruption of the ProSAP2 Gene in a t(12;22)(q24.1;q13.3) is associated with the 22q13.3 Deletion syndrome. Am J Hum Genet. 69:261-268.
Bozdagi O, Sakurai T, Papapetrou D, Wang X, Dickstein DL, Takahashi N, Kajiwara Y, Yang M, Katz AM, Scattoni ML, Harris MJ, Saxena R, Silverman JL, Crawley JN, Zhou Q, Hof PR, Buxbaum JD. (2010) Haploinsufficiency of the autism-associated Shank3 gene leads to deficits in synaptic function, social interaction, and social communication. Mol Autism. 1:15.
Bozdagi O, Tavassoli T, Buxbaum JD. (2013) Insulin-like growth factor-1 rescues synaptic and motor deficits in a mouse model of autism and developmental delay. Mol Autism. 4:9.
Conte MM, Victor JD. VEP indices of cortical lateral interactions in epilepsy treatment. Vision Research. May 2009;49(9):898-906.
Davis S, Vanhoutte P, Pages C, Caboche J, Laroche S. (2000). The MAPK/ERK cascade targets both Elk-1 and cAMP response element-binding protein to control long-term potentiation-dependent gene expression in the dentate gyrus in vivo. J Neurosci. 20(12): 4563-4572.
Duffney LJ, Zhong P, Wei J, Matas E, Cheng J, Qin L, Ma K, Dietz DM, Kajiwara Y, Buxbaum JD, Yan Z. (2015) Autism-like Deficits in Shank3-Deficient Mice Are Rescued by Targeting Actin Regulators. Cell Rep. 11:1400-1413.
Grewal SS, York RD, Stork PJ. (1999) Extracellular-signal-regulated kinase signalling in neurons. Curr Opin Neurobiol. 9:544-553.
Holder J, Quach M. (2016). The spectrum of epilepsy and electroencephalographic abnormalities due to SHANK3 loss-of-function mutations. Epilepsia, 57(10): 1651-1659.
Jaramillo TC, Speed HE, Xuan Z, Reimers JM, Liu S, Powell CM. (2016) Altered Striatal Synaptic Function and Abnormal Behaviour in Shank3 Exon4-9 Deletion Mouse Model of Autism. Autism Res. 9:350-75.
Kolevzon A, Bush L, Wang AT, Halpern D, Frank Y, Grodberg D, Rapaport R, Tavassoli T, Chaplin W, Soorya L, Buxbaum JD. (2014) A pilot controlled trial of insulin-like growth factor-1 in children with Phelan-McDermid syndrome. Mol Autism. 5:54.
Kouser M, Speed HE, Dewey CM, Reimers JM, Widman AJ, Gupta N, Liu S, Jaramillo TC, Bangash M, Xiao B, Worley PF, Powell CM. (2013) Loss of predominant Shank3 isoforms results in hippocampus-dependent impairments in behavior and synaptic transmission. J Neurosci. 33:18448-68.
Lim S, Naisbitt S, Yoon J, Hwang JI, Suh PG, Sheng M, Kim E. (1999) Characterization of the Shank family of synaptic proteins. Multiple genes, alternative splicing, and differential expression in brain and development. J Biol Chem. 274:29510-8.
Nateri A, Gennadij R, Gebhardt C, Da Costa C, Naumann H, Vreugdenhil M, Makwana M, Brandner S, Adams R, Jefferys J, Kann O, Behrens A. (2007). ERK activation causes epilepsy by stimulating NMDA receptor activity. EMBO. 26(23): 4891-4901.
Peça J, Feliciano C, Ting J, Wang W, Wells W, Talaignair T, Lascola C, Fu Z. (2011) Shank3 mutant mice display autistic-like behaviors and striatal dysfunction. Nature. 472 (7344): 437-442.
Phelan MC, Rogers RC, Saul RA et al. (2001) 22q13 Deletion Syndrome. Am. J. Medic. Genet. 101:91-99.
Phelan MC. (2008) Deletion 22q13.3 syndrome. Orphanet J. Rare Dis. 3:14.
Shcheglovitov A, Shcheglovitov O, Yazawa M, Portmann T, Shu R, Sebastiano V, Krawisz A, Freohlich W, Bernstein J, Hallmayer J, Dolmetch R. (2013) SHANK3 and IGF1 restore synaptic deficits in neurons from 22q13 deletion syndrome patients. Nature. 503: 267-271.
Soorya L, Kolevzon A, Zweifach J, Lim T, Dobry Y, Schwartz L, Frank Y, Wang A, Cai G, Parkhomenko E, Halpern D, Grodberg D, Anagartia B, Willner J, Yang A, Canitano R, Chaplin W, Betancur C, Buxbaum J. (2013) Prospective investigation of autism and genotype-phenotype correlations in 22q13 deletion syndrome and SHANK3 deficiency. Mol. Autism. 4: 1-18.
Uppal N, Puri R, Yuk F, Janssen WG, Bozdagi-Gunal O, Harony-Nicolas H, Dickstein DL, Buxbaum JD, Hof PR. (2015) Ultrastructural analyses in the hippocampus CA1 field in Shank3-deficient mice. Mol Autism. 6:41.
Watt JL, Olson, IA, Johnston AW et al. (1985) A familial pericentric inversion of chromosome 22 with a recombinant subject illustrating a "pure" partial monosomy syndrome. J. Med. Genet. 22: 283-287.
Wilson H, Wong A, Shaw S, Tse W, Stapleton G, Phelan M, Hu S, Marshall J, McDermid H. (2003) Molecular characterization of the 22q13 deletion syndrome supports the role of the halpoinsufficiency of SHANK3/PROSAP2 in the major neurological symptoms. J Med Genet. 40:474-584.
Zemon V, Kaplan E, Ratliff F. Bicuculline enhances a negative component and diminishes a positive component of the visual evoked cortical potential in the cat. Proceedings of the National Academy of Sciences of the United States of America. Dec 1980;77(12):7476-7478.
Zemon V, Kaplan E, Ratliff F. Evoked Potentials. In: Cracco RQ, Rodis-Wollner I, eds. Frontiers of Clinical Neuroscience. Vol 3. New York: Alan R. Liss; 1986:287-295.

## Claims

1. A therapeutically effective amount of (i) at least one compound of Formula I or (ii) a pharmaceutical formulation comprising at least one compound of Formula I and a pharmaceutically acceptable carrier or diluent for use in the treatment of Phelan McDermid Syndrome (PMS) in a subject having PMS or for use in a method of treating PMS in a subject having PMS 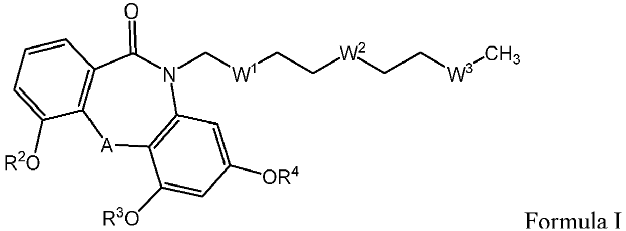 wherein,
each of W¹, W² and W³ is independently 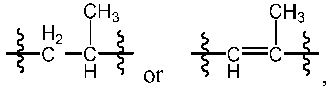 or the chain from the tricycle may terminate at W³, W² or W¹ with W³, W² or W¹ respectively being either -CH=O or -CH₂OH;
A is -NH- or -NC(O)R¹-, where R¹ is C₁₋₆ alkyl;
each of R², R³, and R⁴ is independently H.

2. A therapeutically effective amount of (i) at least one compound of Formula I or (ii) a pharmaceutical formulation comprising at least one compound of Formula I and a pharmaceutically acceptable carrier or diluent for use in the inhibition of development of Phelan McDermid Syndrome (PMS) in a subject predisposed to develop PMS or for use in a method of inhibiting development of PMS in a subject predisposed to develop PMS 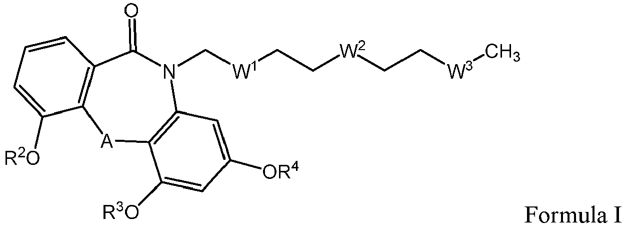 wherein,
each of W¹, W² and W³ is independently 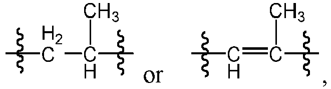 or the chain from the tricycle may terminate at W³, W² or W¹ with W³, W² or W¹ respectively being either -CH=O or -CH₂OH;
A is -NH- or -NC(O)R¹-, where R¹ is C₁₋₆ alkyl;
each of R², R³, and R⁴ is independently H.

3. The therapeutically effective amount for use of claim 1 or 2, wherein the at least one compound of Formula I is AMO-01 or a pharmaceutically acceptable salt thereof.

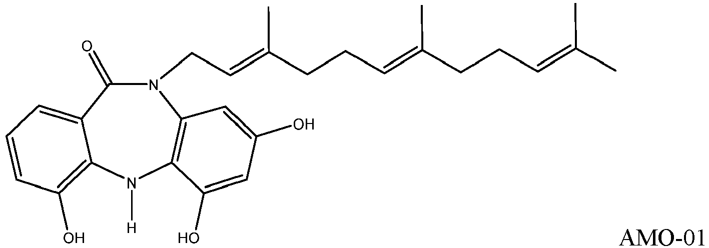

4. The therapeutically effective amount for use of claim 1 or 2, wherein the at least one compound of Formula I is Formula III or a pharmaceutically acceptable salt thereof.

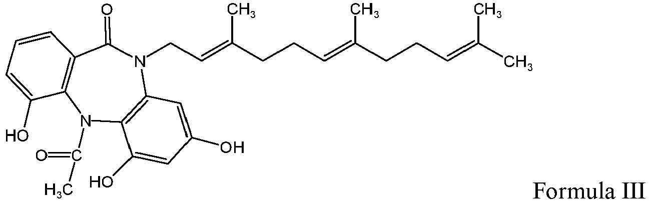

5. The therapeutically effective amount for use of any one of claims 1-4, wherein the subject having PMS or the subject predisposed to develop PMS is a subject having a chromosomal deletion at 22q13.3.

6. The therapeutically effective amount for use of any one of claims 1-5, wherein the therapeutically effective amount of the at least one compound of Formula I is between 0.1 kg and 200 mg/kg of the compound of Formula I per body weight of the subject.

7. The therapeutically effective amount for use of any one of claims 1-5, wherein the therapeutically effective amount of the at least one compound of Formula I is between 80 and 160 mg/m².

8. The therapeutically effective amount for use of any one of claims 1-5, wherein the therapeutically effective amount of the at least one compound of Formula I is 120 mg/m².

9. The therapeutically effective amount for use of any one of claims 1-8, wherein the at least one compound of Formula I or the pharmaceutical formulation comprising at least one compound of Formula I and a pharmaceutically acceptable carrier or diluent is administered to the subject via intravenous or subcutaneous administration.

## Patentansprüche

1. Therapeutisch wirksame Menge (i) mindestens einer Verbindung von Formel I oder (ii) einer pharmazeutischen Formulierung, die mindestens eine Verbindung von Formel I umfasst, und ein pharmazeutisch annehmbarer Träger oder ein Verdünnungsmittel zur Verwendung in der Behandlung von Phelan-McDermid-Syndrom (PMS) bei einem Subjekt mit PMS oder zur Verwendung in einem Verfahren zum Behandeln von PMS bei einem Subjekt mit PMS 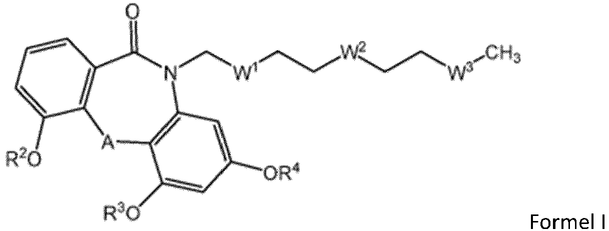 wobei
jedes von W¹, W² und W³ unabhängig 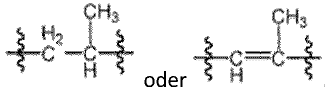 ist, oder die Kette von dem Trizyklus bei W³, W² oder W¹ enden kann, wobei W³, W² oder W¹ jeweils entweder -CH=O oder -CH₂OH sind;
A -NH- oder -NC(O)R¹- ist, wo R¹ C₁₋₆-Alkyl ist;
jedes von R², R³, und R⁴ unabhängig H ist.

2. Therapeutisch wirksame Menge (i) mindestens einer Verbindung von Formel I oder (ii) einer pharmazeutischen Formulierung, die mindestens eine Verbindung von Formel I umfasst, und ein pharmazeutisch annehmbarer Träger oder ein Verdünnungsmittel zur Verwendung in der Hemmung einer Entwicklung von Phelan-McDermid-Syndrom (PMS) bei einem Subjekt, das prädisponiert ist, PMS zu entwickeln, oder zur Verwendung in einem Verfahren zum Hemmen einer Entwicklung von Phelan-McDermid-Syndrom (PMS) bei einem Subjekt, das prädisponiert ist, 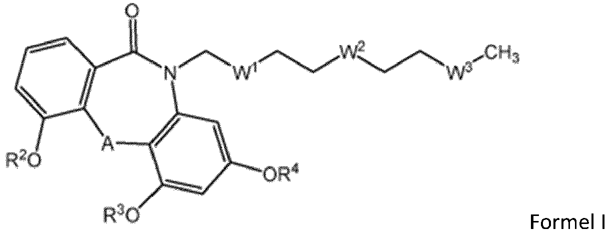 wobei
jedes von W¹, W² und W³ unabhängig 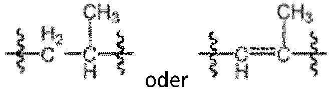 ist oder die Kette von dem Trizyklus bei W³, W² oder W¹ enden kann, wobei W³, W² oder W¹ jeweils entweder -CH=O oder -CH₂OH sind;
A -NH- oder -NC(O)R¹- ist, wo R¹ C₁₋₆-Alkyl ist;
jedes von R², R³, und R⁴ unabhängig H ist.

3. Therapeutisch wirksame Menge zur Verwendung nach Anspruch 1 oder 2, wobei die mindestens eine Verbindung von Formel I AMO-01 oder ein pharmazeutisch annehmbares Salz davon ist.

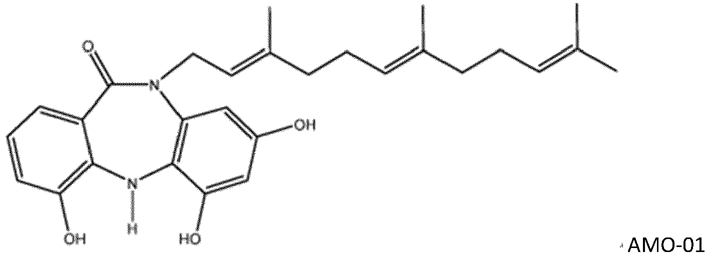

4. Therapeutisch wirksame Menge zur Verwendung nach Anspruch 1 oder 2, wobei die mindestens eine Verbindung von Formel I Formel III oder ein pharmazeutisch annehmbares Salz davon ist.

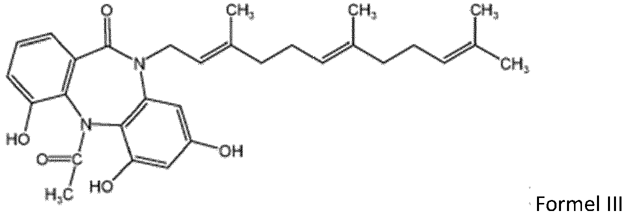

5. Therapeutisch wirksame Menge zur Verwendung nach einem der Ansprüche 1-4, wobei das Subjekt mit PMS oder das Subjekt, das prädisponiert ist, PMS zu entwickeln, ein Subjekt mit einer chromosomalen Deletion bei 22q13.3 ist.

6. Therapeutisch wirksame Menge zur Verwendung nach einem der Ansprüche 1-5, wobei die therapeutisch wirksame Menge der mindestens einen Verbindung von Formel I zwischen 0,1 µg/ kg und 200 mg/kg der Verbindung von Formel I pro Körpergewicht des Subjekts beträgt.

7. Therapeutisch wirksame Menge zur Verwendung nach einem der Ansprüche 1-5, wobei die therapeutisch wirksame Menge der mindestens einen Verbindung von Formel I zwischen 80 und 160 mg/m² beträgt.

8. Therapeutisch wirksame Menge zur Verwendung nach einem der Ansprüche 1-5, wobei die therapeutisch wirksame Menge der mindestens einen Verbindung von Formel I 120 mg/m² beträgt.

9. Therapeutisch wirksame Menge zur Verwendung nach einem der Ansprüche 1-8, wobei die mindestens eine Verbindung von Formel I oder die pharmazeutische Formulierung, die mindestens eine Verbindung von Formel I und einen pharmazeutisch annehmbaren Träger oder ein Verdünnungsmittel umfasst, dem Subjekt durch intravenöse oder subkutane Verabreichung verabreicht wird.

## Revendications

1. Quantité thérapeutiquement efficace de (i) au moins un composé de la Formule I ou (ii) une formulation pharmaceutique comprenant au moins un composé de la Formule I et un excipient ou un diluant pharmaceutiquement acceptable pour son utilisation dans un traitement du syndrome de Phelan McDermid (PMS) chez un sujet présentant le PMS ou pour son utilisation dans une méthode de traitement du PMS chez un sujet présentant le PMS 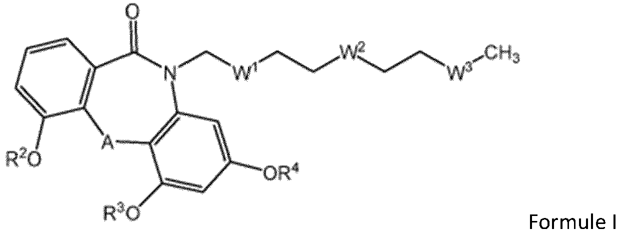 dans laquelle,
chacun de W¹, W² et W³ est indépendamment 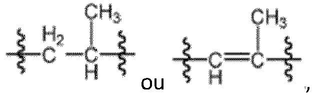 ou la chaîne à partir du tricycle peut se terminer à W³, W² ou W¹ avec W³, W² ou W¹ étant respectivement soit -CH=O soit -CH₂OH ;
A est -NH- ou -NC(O)R¹-, où R¹ est C₁₋₆ alkyl ;
chacun de R², R³, et R⁴ est indépendamment H.

2. Quantité thérapeutiquement efficace de (i) au moins un composé de la Formule I ou (ii) une formulation pharmaceutique comprenant au moins un composé de la Formule I et un excipient ou un diluant pharmaceutiquement acceptable pour son utilisation dans l'inhibition du développement du syndrome de Phelan McDermid (PMS) chez un sujet prédisposé au développement du PMS ou pour son utilisation dans une méthode d'inhibition du développement du PMS chez un sujet prédisposé au développement du PMS 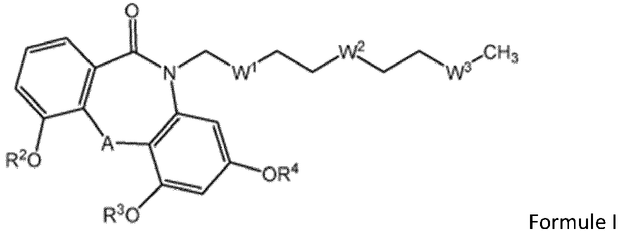 dans laquelle,
chacun de W¹, W² et W³ est indépendamment 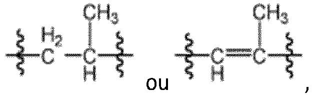 ou la chaîne à partir du tricycle peut se terminer à W³, W² ou W¹ avec W³, W² ou W¹ étant respectivement soit -CH=O soit -CH₂OH ;
A est -NH- ou -NC(O)R¹-, où R¹ est C₁₋₆ alkyl ;
chacun de R², R³, et R⁴ est indépendamment H.

3. Quantité thérapeutiquement efficace pour son utilisation selon la revendication 1 ou 2, dans laquelle l'au moins un composé de la Formule I est AMO-01 ou un sel pharmaceutiquement acceptable de celui-ci.

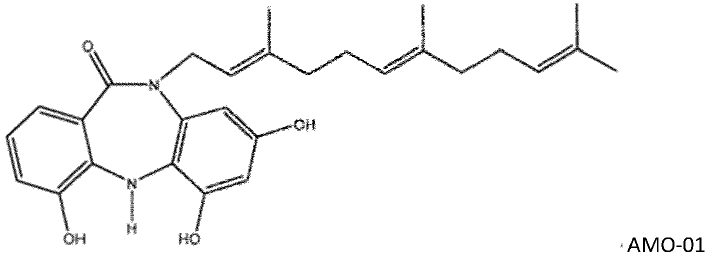

4. Quantité thérapeutiquement efficace pour son utilisation selon la revendication 1 ou 2, dans laquelle l'au moins un composé de la Formule I est la Formule III ou un sel pharmaceutiquement acceptable de celui-ci.

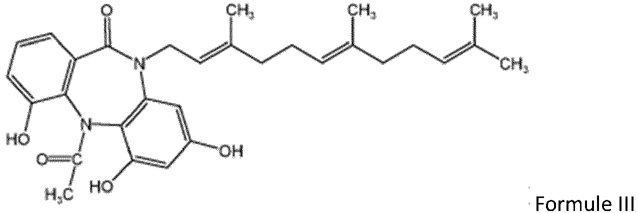

5. Quantité thérapeutiquement efficace pour son utilisation selon l'une quelconque des revendications 1-4, dans laquelle le sujet présentant le PMS ou le sujet prédisposé au développement du PMS est un sujet présentant une délétion chromosomique à 22q13.3.

6. Quantité thérapeutiquement efficace pour son utilisation selon l'une quelconque des revendications 1-5, dans laquelle la quantité thérapeutiquement efficace de l'au moins un composé de la Formule I est entre 0,1 µg/kg et 200 mg/kg du composé de la Formule I par poids corporel du sujet.

7. Quantité thérapeutiquement efficace pour son utilisation selon l'une quelconque des revendications 1-5, dans laquelle la quantité thérapeutiquement efficace de l'au moins un composé de la Formule I est entre 80 et 160 mg/m².

8. Quantité thérapeutiquement efficace pour son utilisation selon l'une quelconque des revendications 1-5, dans laquelle la quantité thérapeutiquement efficace de l'au moins un composé de la Formule I est 120 mg/m².

9. Quantité thérapeutiquement efficace pour son utilisation selon l'une quelconque des revendications 1-8, dans laquelle l'au moins un composé de la Formule I ou la formulation pharmaceutique comprenant au moins un composé de la Formule I et un excipient ou un diluant pharmaceutiquement acceptable est administré au sujet par administration intraveineuse ou sous-cutanée.
